# EUROPEAN PATENT SPECIFICATION

(11) **EP 3 939 602 B1**
(45) Date of publication and mention of the grant of the patent: **29.05.2024**
(21) Application number: 20382632.6
(22) Date of filing: 14.07.2020
(51) Int. Cl.: A61K 35/747, A23L 33/00, A61P 31/00

(54) **LACTOBACILLUS SALIVARUS STRAIN PS11610 AND ITS USE IN THE PREVENTION AND TREATMENT OF UROGENITAL TRACT DYSBIOSIS**
LACTOBACILLUS-SALIVARUS-STAMM PS11610 UND DESSEN VERWENDUNG IN DER VORBEUGUNG UND BEHANDLUNG VON UROGENITALTRAKTDYSBIOSE
SOUCHE DE LACTOBACILLUS SALIVARUS PS11610 ET SON UTILISATION DANS LA PRÉVENTION ET LE TRAITEMENT DE LA DYSBIOSE DU TRACTUS UROGÉNITAL

(43) Date of publication of application: 19.01.2022
(73) Proprietor: Probisearch, S.L.U., 28760 Tres Cantos, Madrid (ES)
(72) Inventor: CÁRDENAS CÁRDENAS, Nivia, E-28760 Tres Cantos, Madrid (ES); ESPINOSA MARTOS, Irene, E-28760 Tres Cantos, Madrid (ES); JIMÉNEZ QUINTANA, Esther Antonia, E-28760 Tres Cantos, Madrid (ES); MANZANO JIMÉNEZ, Susana, E-28760 Tres Cantos, Madrid (ES)
(74) Representative: ABG Intellectual Property Law, S.L.

(56) References cited:
- WO-A1-2017/001440
- WO-A2-2018/013583
- US National Library Of Medicine: "NCT03701893 Evaluate the Use of a New Probiotic Strain in Couples With Fertility Problems and Disbiosis", Clinical Trials Archive, 8 October 2018 (2018-10-08), XP55757869, Retrieved from the Internet: URL:https://clinicaltrials.gov/ct2/history /NCT03701893?V_1=View#StudyPageTop [retrieved on 2020-12-08]
- VIJAY PRABHA ET AL: "Evaluation of profertility effect of probiotic Lactobacillus plantarum 2621 in a murine model", INDIAN JOURNAL OF MEDICAL RESEARCH, vol. 142, no. 1, 1 January 2015 (2015-01-01), page 79, XP055757950, IN ISSN: 0971-5916, DOI: 10.4103/0971-5916.162127

## Description

### FIELD OF THE INVENTION

The invention relates to the field of probiotic strains, in particular to a new probiotic strain of *Lactobacillus salivarius,* to compositions comprising said probiotic strain, and its use for the prevention and/or treatment of urogenital tract dysbiosis.

### BACKGROUND OF THE INVENTION

The urogenital tract contains an active microbiome comprising mainly in the female tract bacteria from the *Lactobacillus* genus, which is associated with a healthy microbiome state. However, fluctuations of this microbiome may impact the physiology of the urogenital tract and even lead to pathological states. Dysbiosis refers to this microbiome imbalance or abnormal microbiota.

Pathogenic organisms are able to infect the vagina, causing bacterial vaginosis and yeast vaginitis. Both bacterial vaginosis and vaginitis in an inflammation of the vagina that can result in discharge, itching and pain.

Bacterial vaginosis represents the most common vaginal syndrome affecting fertile, premenopausal and pregnant women. Bacterial vaginosis is not caused by one specific pathogenic microorganism, but rather by an imbalance of vaginal microbiota. In bacterial vaginosis lactobacilli are replaced by *Gardnerella vaginalis, Atopobium vaginae, Prevotella, Veillonella,* and other anaerobic microorganisms. Bacterial vaginosis is frequently disregarded since the symptoms are often absent or insignificant. The vast majority of cases of bacterial vaginosis do not cause inflammatory symptoms, so it is clinically characterized by an increase in vaginal secretions which makes it more watery and foul-smelling, and which is accompanied by limited additional vulvovaginal symptoms. However, the clinical consequences can be important. In pregnant women, it increases the risk of preterm birth and in non-pregnant women it increases the risk of other infectious diseases, such as herpes, chlamydia and endometritis. It can also directly affect fertility.

Vaginitis is caused by an overgrowth of a fungal organism in the vagina. The majority of cases are caused by *Candida albicans,* but *C. glabrata, C. krusei,* and *C*. *tropicalis* can be problematic. It usually causes many symptoms: whitish vaginal secretion with lumps, pruritus, burning, vulvar inflammation, reddened vaginal mucosa, skin lesions in the vulva and perineum, and sometimes also discomfort when urinating and when having sexual intercourse.

Microbiota of the male genital tract has been disclosed to have an impact in semen quality and, consequently, is related to fertility [Moretti et al., J Assist Reprod Genet2009, 26, 47-56; Mändar, Pharmacol Res., 2013, 69(1), 32-41; and Weng et al., PLoS One, 2014, 9(10), e110152 2014]

The male and female urogenital tract microbiota has a relevant role in infertility [Moreno and Simon, Reprod Med Biol, 2018, 18(1), 40-50]. Thus, treating male and/or female urogenital tract dysbiosis improves the reproductive function in couples suffering from idiopathic infertility.

Current treatments of urogenital tract dysbiosis, including bacterial vaginosis and vaginitis, involves oral or local administration of antibiotics, such as metronidazole or clindamycin, or antifungals. However, there are several unpleasant side effects and disadvantages associated with these therapies, including antibiotic resistance, high rate of recurrent infections after treatment, and side effects derived from the clearance of endogenous off-target flora in other body sites.

Another possible strategy to modulate the urogenital tract microbiota is the use of probiotics. These are live biotherapeutic products containing one or more strains of the desired bacteria that are administered to colonize the niche while displacing potential dysbiotic bacteria. Lactobacilli are the organisms commonly used as probiotics. However, the efficacy of a probiotic therapy is not certain and it is critical that strains are carefully selected [Cribby et al., Interdisciplinary Perspectives on Infectious Diseases, 2008, Article ID 256490; and Mastromarino et al., Indian J. Med. Res., 2014, 140 (Suppl 1), S91-S97].

Vijay Prabha et al. (Indian J. of Med, Res., 2015, 142(1), 79) describe the effect of *Lactobacillus plantarum* 2621 probiotics on fertility and urogenital infections.

WO 2018/013583 A2 refers to the synergic effect of compositions comprising a mixture of *L. crispatus, L. gasseri* and *L. jensenii* in the treatment/prevention of vaginal bacterial infections, however this document mentions that not all *of Lactobacillus* species have the same effect and impact on the vaginal immunobiome.

WO 2017/001440 A1 discloses the use of *Lactobacillus rhamnosus* for the treatment of bacterial vaginosis.

Thus, there is a need for new treatments of urogenital tract dysbiosis, in particular, probiotic treatments.

### BRIEF DESCRIPTION OF THE INVENTION

The invention is solely defined by the appended claims.

The inventors have isolated and characterized a probiotic strain of *Lactobacillus salivarius* (PS 11610) isolated from the vagina of a healthy woman, which has surprisingly been found to be useful for restoring urogenital tract microbiota in subjects with bacterial dysbiosis, in particular in couples with fertility problems.

As shown in the examples of the present invention, *Lactobacillus salivarius* PS 1161 0 affects the bacterial composition of the urogenital male and female tracts improving the dysbiosis status and the immune status of subjects suffering from by bacterial dysbiosis. It has also improved the pregnancy success in couples with idiopathic infertility suffering from bacterial dysbiosis. As also shown in the examples of the present invention, *Lactobacillus salivarius* PS11610 has surprisingly high antimicrobial activity against pathogens related to urogenital tract infections when compared to other *Lactobacillus salivarius* strains. Therefore, the *Lactobacillus salivarius* PS1 1610 strain of the present inventions is particularly suitable for the treatment of dysbiosis of the urogenital tract and related diseases.

Thus, in one aspect, the present invention is directed to a strain of *Lactobacillus salivarius* PS11610 or a variant thereof having at least 99% identity with the 16S rRNA sequence of this strain, and wherein the variant strain retains or further improves the activity of the strain *Lactobacillus salivarius* PS11610 in one or more of the following properties:
- production of lactic acid in MRS broth after 24 h at 37 °C under anaerobic conditions;
- tolerance to pH in the range of 3 to 6; and
- tolerance to bile salts.

Preferably the strain has at least 99.5% identity with the strain of *Lactobacillus salivarius* PS11610.

In another aspect, the invention also relates to a composition comprising the strain as defined above, in particular to a pharmaceutical or food composition.

In further aspect, the invention also relates to the strain, composition or food composition as defined above for use as a probiotic or for use as a therapeutic agent.

In another aspect, the invention is directed to the strain, composition or food composition as defined above, for use in the prevention and/or treatment of urogenital dysbiosis.

In another aspect, the invention is directed to the strain, composition or food composition as defined above, for use in the prevention and/or treatment of bacterial vaginosis and vaginitis.

In another aspect, the invention is directed to the strain, composition or food composition as defined above, for use in the treatment of infertility in subjects suffering from urogenital bacterial dysbiosis.

In an additional aspect, the invention is directed to the strain, composition or food composition as defined above, for use in reducing pathogenic bacteria in the urogenital tract.

### FIGURES

Figure 1 shows the growth curve of *L. salivarius* PS11610.
Figure 2 shows the survival of *L. salivarius* PS11610 in MRS acidified at pH 6, 4, 3.5 and 3 after 2 h incubation at 37°C in aerobiosis. Striped bars (time 0 h) and unstriped bars (time 2 h).
Figure 3 shows the survival of *L. salivarius* PS1 1610 in MRS with 0.2, 0.4 and 0.5 % bile after 24 h incubation at 37°C in aerobiosis. Striped bars (time 0 h) and unstriped bars (time 24 h).
Figure 4 shows the evolution in bacterial counts in vaginal samples due to intake of *L. salivarius* PS11610.

### DETAILED DESCRIPTION OF THE INVENTION

### Lactobacillus salivarius strain PS1 1610

The strain of *Lactobacillus salivarius* according to the invention is referred to as *Lactobacillus salivarius* PS11610 or *L. salivarius* PS11610. The strain *L. salivarius* PS11610 has been deposited by Probisearch SLU, Calle Santiago Grisolia 2, Tres Cantos, Spain in accordance with the Budapest Treaty on the International Recognition of the Deposit of Microorganisms for the Purpose of Patent Procedure at the Colección Española de Cultivos Tipo (CECT) on 13 December 2017 and has been given Accession Number CECT 9526.

The term "strain" is well-known in the field and means a genetic variant or subtype of a microorganism. A "variant" or "mutant" of a strain refer to any naturally-occurring or specifically developed strain obtained from the reference strain *L. salivarius* PS11610, mainly by mutation, that maintains or enhances the properties of the reference strain. Variant strains may be obtained by using the deposited strain as starting material. Thus, in the present case, the invention relates to variants of the reference strain in which antimicrobial activity against one or more of *Candida albicans, Actinomyces neuii, Aerococcus urinae, Actinomyces urogenitalis, Gardnerella vaginalis, Klebsiella oxytoca, Streptococcus agalactiae, Escherichia coli, Klebsiella pneumoniae, Serratia marcescens, Cronobacter sakazakii, Enterococcus faecalis, Staphylococcus aureus, Staphylococcus epidermidis* and *Listeria innocua;* and in which one or more of the following properties associated to the *L. salivarius* PS11610 strain is preserved or enhanced:
- production of lactic acid in MRS broth after 24 h at 37 °C under anaerobic conditions;
- tolerance to pH in the range of 3 to 6; and
- tolerance to bile salts.

As used herein, the reference to that the variant strain maintains or preserves the properties of the reference strain *L. salivarius* PS1 1610 means that, while not showing an activity of 100% of the property of the reference strain *L. salivarius* PS 11610 from which it derives, the property is substantially preserved so that the variant shows at least 99%, at least 98%, at least 97%, at least 96%, at least 95%, at least 94%, at least 93%, at least 92%, at least 91%, at least 90%, at least 80%, at least 70%, at least 60%, at least 50% of the property of the *L. salivarius* PS1 1610 strain from which it derives.

The person skilled in the art will decide upon the adequate method to be employed for determining each of the properties mentioned above for the strains, in particular using the methods described in the examples of the present application.

The present invention also relates to strains of *L. salivarius* having at least 99 % identity with the 16S rRNA sequence of the *L. salivarius* PS11610 strain, which is described in the examples [reference Stackebrandt & Goebel, 1994: "Taxonomic Note: A Place for DNA-DNA Reassociation and 16s rRNA Sequence Analysis in the Present Species Definition in Bacteriology" Int. J. Syst. Bacteriol. 44:846-849].

In a preferred embodiment, the strain according to the present invention has at least 99.5 % identity with the 16S rRNA sequence of the *L. salivarius* PS11610 strain, more preferably at least 99.9% identity. In another preferred embodiment, the strain according to the present invention has 100% identity with the 16S rRNA sequence of the *L. salivarius* PS11610 strain, i.e. the strain according to the present invention is the *Lactobacillus salivarius* PS11610 strain.

The identity of the variant strain with respect to the *L. salivarius* PS1 1610 strain may be determined as "average nucleotide identity" or "ANI", which refers to a measure of the nucleotide-level genomic similarity between the coding regions of two genomes. The term ANI includes:
- Average nucleotide identity based on BLAST+ (ANIb), described by Richter M, Rosselló-Móra R, Glöckner FO, and Peplies J (2015) JSpeciesWS: a web server for prokaryotic species circumscription based on pairwise genome comparison. Bioinformatics. 2015 Nov 16. pii: btv681.
- Average nucleotide identity based on MUMmer (ANIm), also described by Richter M, Rosselló-Móra R, Glöckner FO, and Peplies J (2015) JSpeciesWS: a web server for prokaryotic species circumscription based on pairwise genome comparison. Bioinformatics. 2015 Nov 16. pii: btv681.
- Ortho ANI using BLAST (orthoANIb) and orthoANI using USEARCH (orthoANUu), described by Yoon, S. H., Ha, S. M., Lim, J. M., Kwon, S.J. & Chun, J. (2017). A large-scale evaluation of algorithms to calculate average nucleotide identity. Antonie van Leeuwenhoek. 110:1281-1286.

The strain of the present invention may be alive or inactivated.

Alive microorganisms produce a complete array of antigens, reproduce to increase the number of such organisms in the urogenital environment to better stimulate an immune response. Thus, in a preferred embodiment, the strain of the invention is alive.

As used herein, the term "inactivated" refers to a dead or inactivated cell of a microorganism which is no longer capable to form a single colony on a plate having medium specific for said microorganism, and it also encompasses lysates, fractions or extracts of the microorganism. The inactivation of a cell refers to a process of transforming a cell from viable to non-viable. The terms "viable", "alive" or "viability", as used herein, refer to the ability of a cell to maintain itself or recover its potentialities and survive until they are able to divide. Thus, a cell that is viable or alive indicates that the cell is able to survive and divide; conversely, a cell that is non-viable indicates that the cell is not able to survive and divide. It will be appreciated that non-viable cells include dead cells. Thus, in another embodiment, the strain of the invention is inactivated.

Viability can be determined by a number of assays that are conventional in the art. These include cytolysis or membrane leakage assays, such as the lactate dehydrogenase assay, the propidium iodide assay, the trypan blue assay and the 7-aminoactinomycin D assay, as well as genomic and proteomic assays that test the activation of stress pathways using DNA microarrays and protein chips. Viability can also be determined by checking the absence of cells after their culture in an appropriate culture medium.

### Compositions

In another aspect, the present invention relates to a composition comprising the *L. salivarius* PS1 1610 strain of the invention, or variant thereof.

Preferably, the compositions of the invention comprises the *L. salivarius* PS 11610 strain of the invention or variant thereof and a physiologically acceptable carrier or excipient and/or further ingredients as described further below. Preferably, the *L. salivarius* strain according to the invention is present in freeze-dried form.

The term "composition", as used in the present invention relates to any composition of matter comprising the strain of the invention, i.e. *L. salivarius* PS11610 strain or variant thereof.

In a preferred embodiment, the composition of the invention comprises the *L. salivarius* PS11610 strain or variant thereof and a pharmaceutically acceptable excipients. These compositions can also be named as "pharmaceutical composition" or "pharmaceutical product".

As used in the present invention, the expressions "pharmaceutical composition" or "pharmaceutical product" are used herein interchangeably and refer to a formulation which has been adapted to administer a predetermined dose of one or several therapeutically useful agents to a subject, preferably a human being. The pharmaceutical composition of the invention contains a therapeutically effective amount of the *Lactobacillus salivarius* PS1 1610 strain of the invention or a variant thereof, which is understood herein as an amount capable of providing a therapeutic effect, and which can be determined by the person skilled in the art by commonly used means.

The "pharmaceutical acceptable excipient" refers to a diluent or carrier with which the active ingredient, i.e. the strains of the invention, is administered. Acceptable excipients for therapeutic use are well known in the pharmaceutical art, and are described, for example, in Remington: The Science and Practice of Pharmacy, Lippincott Williams and Wilkins (22nd Ed., 2012). The choice of pharmaceutical excipient can be selected with regard to the intended route of administration and standard pharmaceutical practice. For example, such pharmaceutical excipients include, but are not limited to, maltodextrin, water, glucose, lactose, sucrose, mannitol, starch, cellulose or cellulose derivatives, e.g. methylcellulose, magnesium stearate, stearic acid, sodium saccharin, talcum, magnesium carbonate and the like; preferably maltodextrin. Water or aqueous solutions of saline solution and aqueous dextrose and glycerol solutions are preferably used as excipients. The pharmaceutical compositions of the invention may comprise as - or in addition to - the excipients mentioned above any suitable binder(s), lubricant(s), suspending agent(s), coating agent(s), solubilizing agent(s). Preservatives, stabilizers, dyes and even flavoring agents may be provided in the pharmaceutical compositions. Examples of preservatives include sodium benzoate, sorbic acid and esters of p-hydroxybenzoic acid. Antioxidants and suspending agents may also be used. There may be different composition/formulation requirements depending on the different delivery systems.

The compositions of the invention, in particular pharmaceutical compositions, are preferably in the form of an oral formulation, such as capsules, sachets, tablets, granules, powders, pellets, syrups, and suspensions, preferably capsules or sachets, more preferably capsules.

In a preferred embodiment, the composition of the invention comprises maltodextrin as the pharmaceutically acceptable excipient.

In another preferred embodiment, the composition of the invention comprises maltodextrin as the only excipient. Thus, preferably, the composition of the invention consist of the strain of the invention and maltodextrin.

In a particular embodiment, the composition of the invention is a solid form having a weight in the range of from 200 mg to 400 mg, preferably from 250 mg to 350, more preferably from 290 mg to 310 mg, even more preferably about 300 mg. Preferably, said composition is in the form of a capsule or sachet, even more preferably a capsule. Also preferably, said composition comprises the strain of the invention and maltodextrin as the pharmaceutically acceptable excipient.

In a particular embodiment the strain of the invention is present in a foodstuff. These compositions can also be named as terms "food product" or "food composition". These compositions relate to a food that beneficially affects one or more functions of the body, so as to provide better health and wellness. Accordingly, such a food product may be intended for the prevention and/or treatment of a disease or a disease causing factor. Therefore, these compositions can also be named as functional food for particular nutritional purposes.

Non-limiting examples of suitable foodstuffs which can be used in the present invention are dairy products, bread, cakes, semi- or synthetic diet formulations, infant formulae, clinical nutrition formulae, flours.

The effective amount of colony forming units (CFU) for each strain in the composition of the invention will be determined by the skilled person and will depend upon the final formulation and the times per day it will be administered. Preferably, the strain of the invention is present in the compositions of the invention in an amount from about 10⁶ CFU to about 10¹² CFU, preferably in an amount from about 10⁷ CFU to about 10¹¹ CFU, more preferably in an amount from about 10⁸ CFU to about 10¹⁰ CFU, even more preferably about 10⁹ CFU.

In the context of the present invention, the term "about" refers to the value it defines ±10% of said value, preferably ±5% of said value, more preferably ±1% of said value.

The term CFU refers to "Colony Forming Units". Quantification of bacteria in a given sample is routinely achieved by counting the total number of colony-forming units (CFUs) grown on an agar plate from serial dilutions, expressed as CFU per gram or mL of the original sample. This yields an estimate of the number of cells present based on a skilled interpretation of the number of colonies on a plate.

A particularly preferred composition of the invention comprises the *Lactobacillus salivarius* PSI 1610 strain of the invention and maltodextrin. Preferably, the composition comprises the *Lactobacillus salivarius* PS 11610 strain of the invention and maltodextrin, wherein the amount of the *Lactobacillus salivarius* PS11610 strain of the invention is from about 10⁶ CFU to about 10¹² CFU, preferably in an amount from about 10⁷ CFU to about 10¹¹ CFU, more preferably in an amount from about 10⁸ CFU to about 10¹⁰ CFU, even more preferably about 10⁹ CFU.

The composition according to the invention may comprise further probiotics, apart from the *L. salivarius* PS1 1610 strain of the invention or a variant thereof as described above. Probiotics have beneficial effects on the immune system, hence the combination with probiotics will have a superior effect on immune system. Examples of further probiotics are *Lactobacillus* and *Bifidobacterium.*

The composition of the present invention may further contain prebiotics. Prebiotics may support the growth of probiotics before they are rendered non-replicating. "Prebiotic" means non-digestible food substances that promote the growth of health beneficial microorganisms and/or probiotics in the intestines. They are not broken down in the stomach and/or upper intestine or absorbed in the GI tract of the person ingesting them, but they are fermented by the gastrointestinal microbiota and/or by probiotics. Preferably, they may be selected from the group consisting of oligosaccharides, optionally containing fructose, galactose, mannose; dietary fibers, in particular soluble fibers, soy fibers; inulin; or mixtures thereof. Preferred prebiotics are fructo-oligosaccharides, galacto-oligosaccharides, isomalto-oligosaccharides, xylo-oligosaccharides, arabino-xylo oligosaccharides, mannan-oligosaccharides, oligosaccharides of soy, glycosyl sucrose, lactosucrose, lactulose, palatinose-oligosaccharides, malto-oligosaccharides, gums and/or hydrolysates thereof, pectins and/or hydrolysates thereof.

### Uses of the strain and composition of the invention

As evidenced by the examples below, the strains and compositions of the invention are safe and have an important beneficial effect. In an *in vivo* human study, the strain of the invention has improved the dysbiosis status and the immune status of the urogenital tract in both women and men. In particular the strain of the invention has improved the pregnancy success in couples with idiopathic infertility suffering from bacterial dysbiosis.

Thus, in one aspect, the present invention is directed to a strain of *Lactobacillus salivarius* PS11610 or a variant thereof having at least 99% identity with this strain, and wherein the variant strains retain or further improve the activity of the strain *Lactobacillus salivarius* PS11610. Preferably the strain has at least 99.5% identity with the strain of *Lactobacillus salivarius* PS11610.

Thus, in further aspect, the invention also relates to the strain, the composition or the foodstuff as defined above for use as a probiotic or for use as a therapeutic agent.

This aspect may also be formulated as the use of the strain, the composition or the foodstuff as defined above in the manufacture of a probiotic or of a medicament.

In the present invention, the term "probiotic" means a microorganism that exerts beneficial effects on the health of the host. It can be a live microbial fed supplement or medicament that beneficially affects the host by improving its microbial balance, a microbial preparation that contains live or dead bacteria, or a combination of both. Hosts suitable in the context of the invention includes human beings.

As used herein the terms "treat", "treatment", or "treatment of" refers to reducing the potential for a certain disease or disorder, reducing the occurrence of a certain disease or disorder, and/or a reduction in the severity of a certain disease or disorder, preferably, to an extent that the subject no longer suffers discomfort and/or altered function due to it. It also refers to mitigating or decreasing at least one clinical symptom and/or inhibition or delay in the progression of the condition and/or prevention or delay of the onset of a disease or illness.

The term "prevention", "preventing" or "prevent", as used herein, refers to avoiding the appearance of a certain disease or disorder. The prevention can be complete (e.g. the total absence of a disease). The prevention can also be partial, such that for example the occurrence of a disease in a subject is less than that which would have occurred without the administration of the combination or composition of the present invention. Prevention also refers to reduced susceptibility to a clinical condition. The prevention also includes reducing the risk of suffering the disease.

In another aspect, the invention is directed to the strain, the composition or the foodstuff as defined above, for use in the prevention and/or treatment of urogenital dysbiosis.

This aspect may also be formulated as the use of the strain, the composition or the foodstuff as defined above, in the manufacture of a medicament for the prevention and/or treatment of urogenital dysbiosis.

This aspect may also be formulated as a method of prevention and/or treatment of urogenital dysbiosis comprising administering the strain, the composition or the foodstuff as defined above to a subject in need thereof.

In particular the urogenital dysbiosis refers to dysbiosis in the vagina, uterus, glans and/or semen, preferably vaginal dysbiosis.

The criteria to determine the bacterial urogenital dysbiosis status are:
- bacterial concentration <50 CFU in vaginal exudates;
- lactobacilli concentration in ovulation <10² CFU in vaginal mucosa;
- concentration of corynebacteria, enterococci and / or *Staphylococcus aureus* > 10⁵ CFU in male samples (in particular glans and/or semen); and/or
- presence of *Actinomyces neuii, Gardnerella vaginalis* and/or *Enterobacteriaceae* in any type of urogenital sample.

A subject meeting at least one of the above four criteria is considered to have dysbiosis.

In a particular, the vaginal dysbiosis in related to bacterial vaginosis and vaginitis. Thus, the strain or composition of the invention is for use in the prevention and/or treatment of bacterial vaginosis and/or vaginitis.

This aspect may also be formulated as the use of the strain, the composition or the foodstuff as defined above, in the manufacture of a medicament for the prevention and/or treatment of bacterial vaginosis and/or vaginitis.

This aspect may also be formulated as a method of prevention and/or treatment of bacterial vaginosis and/or vaginitis comprising administering the strain, the composition or the foodstuff as defined above to a subject in need thereof.

In a further aspect, the invention relates to the strain, the composition of the foodstuff as defined above for use in the prevention of miscarriage in the first trimester of pregnancy in *in vitro* fertilization.

This aspect may also be formulated as the use of the strain, the composition or the foodstuff as defined above, in the manufacture of a medicament for the prevention of miscarriage in the first trimester of pregnancy in *in vitro* fertilization.

This aspect may also be formulated as for the prevention of miscarriage in the first trimester of pregnancy in *in vitro* fertilization, comprising administering the strain, the composition or the foodstuff as defined above to a subject in need thereof.

In a further aspect, the invention relates to the strain, the composition of the foodstuff as defined above for use in the treatment of infertility in a subject suffering from urogenital bacterial dysbiosis.

This aspect may also be formulated as the use of the strain, the composition or the foodstuff as defined above, in the manufacture of a medicament for the treatment of infertility in a subject suffering from urogenital bacterial dysbiosis.

This aspect may also be formulated as a method of treatment of infertility comprising administering the strain, the composition or the foodstuff as defined above to a subject in need thereof and suffering from urogenital dysbiosis.

The term "infertility" is defined by the World Health Organization as a disease of the reproductive system defined by the failure to achieve a clinical pregnancy after 12 months or more of regular unprotected sexual intercourse. Infertility can be related to the man, the woman or both members of a couple. In particular, the infertility refers to idiopathic infertility, i.e. unexplained infertility in the sense that the cause that a couple cannot conceive remains unknown.

The subject refers to a woman, a man or both of a couple, preferably a woman.

In an additional aspect, the invention is directed to the strain, the composition or the foodstuff as defined above, for use in reducing pathogenic bacteria in the urogenital tract.

This aspect may also be formulated as the use of the strain, the composition or the foodstuff as defined above, in the manufacture of a medicament for reducing pathogenic bacteria in the urogenital tract.

This aspect may also be formulated as a method of reducing pathogenic bacteria in the urogenital tract comprising administering the strain, the composition or the foodstuff as defined above to a subject in need thereof and suffering from urogenital dysbiosis.

In particular, the pathogenic bacteria may be present in the vagina, glans and/or semen, preferably in the vagina.

The expression "pathogenic bacteria" refers to any bacteria that can cause a disease. Examples of pathogenic bacteria are *Corynebacterium, Enterococcus, S. aureus, Actinomyces neuii, Gardnerella vaginalis* and *Enterobacteria.*

The term "reducing pathogenic bacteria" means that the pathogenic bacterial counts are lower than before administering the strain, composition or foodstuff of the invention. Preferably, the pathogenic bacterial counts are reduced by at least 50%, preferably at least 55%, preferably at least 60%, preferably at least 65%, preferably at least 70% preferably at least 75%, preferably at least 80%, preferably at least 85%, preferably at least 90%, preferably at least 95%, with respect to the pathogenic bacterial counts before administering the strain, composition or foodstuff of the invention. Any suitable method known in the art may be used for determining the bacterial counts.

The strain, composition and foodstuff for use in the present invention described above are preferably administered enterically, more preferably orally. The strain and composition for use according to the invention may be administered in a single daily dose or multiple doses per day, such as at least 2 doses per day, at least 3 doses per day, at least 4 doses per day. Preferably, the strain and composition according to the invention are administered 1 or 2 doses per day. The person skilled in the art will be able to adjust the therapeutically effective dose and/or the prophylactic effective dose appropriately. The precise dose depends on a number of factors such as the mammal's health and weight. Preferably the strain and composition of the invention are administered in an amount that provides a dose of the strain from about 10⁶ CFU/day to about 10¹² CFU/day, preferably in an amount from about 10⁷ CFU/day to about 10¹¹ CFU/day, more preferably in an amount from about 10⁸ CFU/day to about 10¹⁰ CFU/day, even more preferably about 10⁹ CFU/day or about 2·10⁹ CFU/day to the subject receiving said strain, composition of foodstuff. Preferably, when the subject is a woman, she is administered one of the above doses twice daily. Preferably, when the subject is a man, he is administered one of the previously mentioned doses once daily or twice daily. In a particular embodiment, when the subject is a man, he is administered one of the previously mentioned doses once daily. In another particular embodiment, when the subject is a man, he is administered one of the previously mentioned doses twice daily

Preferably, the strain, composition and foodstuff of the invention is administered for about 12 weeks to about 1 year, more preferably for about 12 weeks to about 9 months, still more preferably for about 12 weeks to about 7 months, even more preferably for about 6 months.

### EXAMPLES

### Example 1: Identification and characterization of the strain PS11610

### 1.1. Isolation of Lactobacillus salivarius PS11610 and identification (species and strain)

*Lactobacillus salivarius* PS 1161 0 was isolated from the vagina of a healthy woman. A vaginal swab was aseptically collected in a sterile tube and kept at 4°C until delivery to the laboratory. The biological material contained in the vaginal swab was resuspended in 1 mL of peptone water. Dilutions of the sample with peptone water were plated in triplicate onto de Man, Rogosa, and Sharpe (MRS, Oxoid, Basingstoke, UK) supplemented with L-cysteine (0.5 g L⁻¹) (MRS-C) agar plates, which were incubated anaerobically (85% nitrogen, 10% hydrogen, 5% carbon dioxide) in an anaerobic workstation (MINI-MACS, DW Scientific, Shipley, UK) at 37 °C for 48 h.

The strain was identified at the species level as *Lactobacillus salivarius* by PCR amplification of a section of a 16S rRNA gene variable region using primers pb116 (5'-AGAGTTTGATCCTGGCTCAG-3', SEQ ID NO: 1) and mb116 (5'-GGCTGCTGGCACGTAGTTAG-3', SEQ ID NO: 2) [Kullen et al., Journal of Applied Microbiology, 2000, 89, 511-518]. PCR conditions were as follows: 96 °C for 30 s, 50 °C for 30 s and 72 °C for 45 s (35 cycles) and a final extension at 72 °C for 4 min. Amplified fragments were purified using the NucleoSpin Extract II (Macherey-Nagel Gmb; Düren, Germany) and sequenced using the primers cited above on an ABI 377A automated sequencer (Applied Biosystems, Foster City, USA). The sequences were compared with those deposited in the EMBL database using BLAST algorithm (http://www.ncbi.nlm.nih.gov/BLAST).

The fragment sequence from *L. salivarius* PS11610 16S rRNA gene has SEQ ID NO: 3, which is reproduced below:

AACGTCATCGATGAACAGTTACTCTCACTCGTGTTCTTCTCTAACAACAGAG
TTTTACGATCCGAAGACCTTCTTCACTCACGCGGCGTTGCTCCATCAGACTT
GCGTCCATTGTGGAAGATTCCCTACTGCTGCCTCCCGTAGGAGTTTGGGCCG
TGTCTCAGTCCCAATGTGGCCGATCAACCTCTCAGTTCGGCTACGTATCATC
ACCTTGGTAGGCCGTTACCCCACCAACTAGTTAATACGCCGCGGGTCCATCT
AAAAGCGATAGCAGAACCATCTTTCATCTAAGGATCATGCGATCCTTATAG
ATATACGGGATTAGCACCTGTTTCCAAGTGTTATCCCCTTCTTTTAGGCAGG
TTACCCACGTGTTACTCACCCGTCCGCCACTCAACTTCTTACGGTGAATGCA
AGCATTCGGTGTAAGAAAGTTTCGTTCGACTTGCATGTATTAGGCACGCCGC
CAGCGTTCGTCCTGAGCCAGGAGCAAACTCTCA

The identification was confirmed by Matrix Assisted Laser Desorption Ionization-Time of Flight (MALDI-TOF) mass spectrometry using a Vitek-MS^{™} instrument (BioMérieux, Marcy l'Etoile, France) in Probisearch (Tres Cantos, Spain). Briefly, a portion of a bacterial colony (~1 µL) was directly spotted onto a MALDI sample plate. Then, it was overlaid with 1 µL of a saturated solution of α-cyano-4-hydroxycinnamic acid in acetonitrile (28%) and allowed to dry at room temperature. A mean spectrum was constructed with at least 50 m/z spectra profiles and used for the identification by comparison with the spectra contained in the Myla database (Biomerieux). Identification was defined as a 99-100% match to the species-specific m/z values in the database.

This strain has been genotyped by pulsed-field gel electrophoresis (PFGE) analyses.

The complete genome of the strain *L. salivarius* PS11610 has been sequenced and analyzed.

### 1.2. Carbohydrate fermentation and enzymatic patterns

The carbohydrate fermentation profile was determined by using the API 50 CH system (BioMérieux, Lyon, France) according to the manufacturer's instructions. The test was carried out from a pure culture in MRS broth with an OD ₅₅₀ₙₘ 0.5. In order to obtain a cells, the culture was centrifuged at 3980× g for 10 min, at 4°C and and resuspended in API 50 CHL. The enzymatic activitiy profile was assayed by using the API Zym galleries (BioMérieux) following the manufacturer's instructions. The cells obtained from culture of OD ₅₅₀ₙₘ 1.2, were resuspended in 0.85 % (w/v) NaCl.

*L. salivarius* PS11610 produced acid from the following substrates: D-Galactose, D-Glucose, D-Fructose, D-Mannose, D-Mannitol, D-Sorbitol, N-Acetylglucosaminose, Amygdalin, D-Maltose, D-Lactose, D-Melibiosa, D-Sucrose, D-Trehalose, D-Rafinosa. Furthermore, this strain displayed the following enzymatic activities: Alkaline phosphatase, Esterase (C4), Esterase Lipase (C8), Lipase (C14), Leucine arylamidase, Valine arylamidase, Cystine arylamidase, Acid phosphatase, Naphthol-AS-BI-phosphohydrolase and α-Galactosidase (Table 1).

**Table 1. Phenotypic characteristics of L. salivarius PS11610**

| **Test** | **Reaction or characteristic** | **Test** | **Reaction or characteristic** |
|---|---|---|---|
| Morphology | Rod | D-Raffinose | + |
| Gram reaction | + | Starch | - |
| Catalase | - | Glycogen | - |
| Oxidase | - | Xylitol | - |
| **Fermentation of** | | β-Gentibiose | - |
| Glycerol | - | D-Turanose | - |
| Erythritol | - | D-Lyxose | - |
| D-Arabinose | - | D-Tagatose | - |
| L-Arabinose | - | D-Fucose | - |
| Ribose | - | L-Fucose | - |
| D-Xylose | - | D-Arabitol | - |
| L-Xylose | - | L-Arabitol | - |
| Adonitol | - | Gluconate | - |
| β-Methylxyloside | - | 2-Ketogluconate | - |
| Galactose | + | 5-Ketogluconate | - |
| D-Glucose | + | Inulin | - |
| D-Fructose | + | **Enzymatic activities:** | |
| D-Mannose | + | Alkaline phosphatase | + |
| L-Sorbose | - | Esterase (C4) | + |
| Rhamnose | - | Esterase-lipase (C8) | + |
| Dulcitol | - | Lipase (C14) | + |
| Inositol | - | Leucine arylamidase | + |
| Mannitol | + | Valine arylamidase | + |
| Sorbitol | + | Cistine arylamidase | + |
| α-Methyl-d-mannoside | - | Tripsym | - |
| a -Methyl-d-glucoside | - | α-quimotripsyn | - |
| N-acetylglucosamine | + | Acid phosphatase | + |
| Amygdalin | + | Naphthol-AS-BI-phosphohydrolase | + |
| Arbutin | - | α-Galactosidase | + |
| Esculin | - | β-Galactosidase | - |
| Salicin | - | β-Glucuronidase | - |
| Maltose | + | α-Glucosidase | - |
| Lactose | + | β-Glucosidase | - |
| Melibiose | + | *N*-acetyl-β-glucosaminidase | - |
| Sacarose | + | α-Manosidase | - |
| Trehalose | + | α-Fucosidase | - |
| Melezitose | - | | |

### 1.3. Antibiogram

Minimum inhibitory concentrations (MIC) for the antibiotics Ampicillin, Clindamycin, Chloramphenicol, Erythromycin, Streptomycin, Gentamicin, Kanamycin, Tetracycline and Vancomycin were determined by a microdilution method. *L. salivarius* PS 11610 was culture in lactic acid bacteria susceptibility test medium (LSM) [Klare et al., Applied and Environmental Microbiology, 2005, 71, 8982-8986] and diluted to obtain a density corresponding to McFarland standard 1 (spectrophotometric equivalent ~ 10⁸ CFU mL⁻¹). The suspension was further adjusted to 10⁵ CFU mL⁻¹ with LSM, and 100 µL were inoculated to wells of microtiter VetMIC plates for lactic acid bacteria (National Veterinary Institute of Sweden, Uppsala, Sweden). MIC was defined as the lowest concentration at which no growth was observed after incubated the plate at 37 °C in aerobic conditions for 48 h. MICs values of *L. salivarius* PS 11610 were compared with the microbiological cut-off parameters established by the European Food Safety Authority (EFSA, 2017) for homofermentative *Lactobacillus.*

According to these values, this lactobacillus strain was sensitive to all the antibiotics tested in this study with the exception of kanamycin (a fact that was expected since all the strains of the *L. salivarius* species are intrinsically resistant to these two antibiotics) (Table 2).

**Table 2. Minimum inhibitory concentrations (MIC) and cut-off values (µg mL⁻¹) of antibiotics against L. salivarius PS11610.**

| **Antibiotics** | **Cut-off values*** | **MIC (*L. salivarius* PS11610)** |
|---|---|---|
| Ampicillin | 4 | 0.5 |
| Clindamycin | 4 | 0.5 |
| Chloramphenicol | 4 | 2 |
| Erythromycin | 1 | 0.12 |
| Streptomycin | 64 | 32 |
| Gentamicin | 16 | 4 |
| Kanamycin | 64 | 128 |
| Tetracyclin | 8 | 2 |
| Vancomycin | n.r. | >128 |

| | | |
|---|---|---|
| *EFSA (2017) n.r.: not required | | |

### 1.4. Evaluation of the L. salivarius PSI 1610 growth rate

To evaluate the ability of *L. salivarius* PS1 1610 of growing in aerobic conditions, bacterial strain was cultured in 120 mL of MRS broth (0.1%) at 37 °C for 24 h. Each 2 h, during the next 24 h, OD₆₀₀, pH and bacterial counts were registered, in duplicate. Viable bacterial counts were determined by plating adequate dilutions on MRS agar plates and incubating as mentioned above.

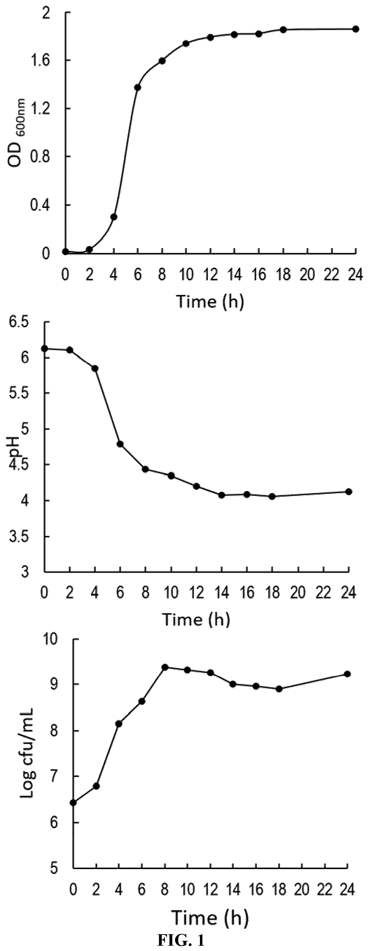

As shown in Figure 1, *L. salivarius* PS11610 is able to reach the stationary phase after incubation at 37 °C in aerobiosis in 6 hours. Also *L. salivarius* PS1 1610 exhibited the highest viable counts (9.38 log₁₀ CFU mL⁻¹) after 8 h. In addition, it is able to rapidly acidify the medium, in which it grows, reaching pH values of 4.3 after 8 hours of growth.

### 1.5. Acid tolerance

The acid tolerance of the *L. salivarius* PS11610 was determined in MRS broth adjusted to pH 6, 4, 3.5 or 3 (using HCl, 5N). Initial MRS had an initial pH of 6.6 and it was used as control. After inoculation with a fully-grown culture (1%, v/v), the mixtures were incubated at 37 °C for 2 h and the viable cells were determined by plate counting.

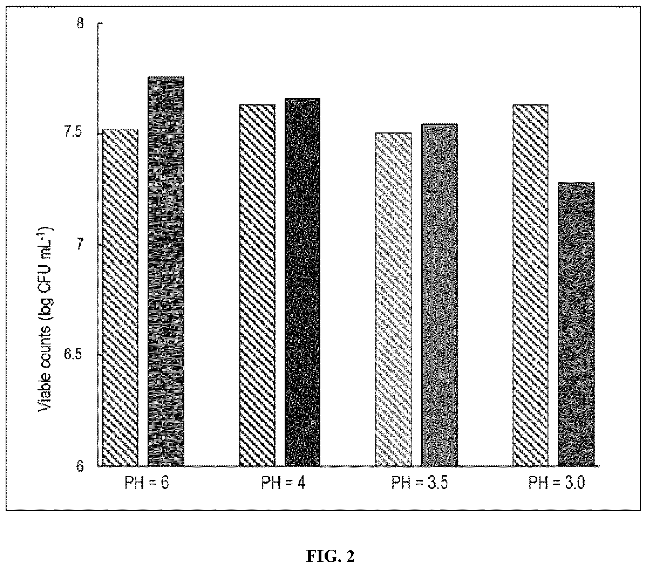

*L. salivarius* PS11610 showed excellent tolerance to the acidity conditions tested (Figure 2). The viable counts increased by about 0.5 log units in MRS pH 4 and pH 3.5 after 2 h of incubation. Furthermore, it maintained a high viable count (7.28 log CFU mL⁻¹) under the most extreme condition tested (pH 3).

### 1.6. Bile tolerance

The survival in the presence of bile salts of *L. salivarius* PS1 1610 was determined in MRS broth (24 h, aerobiosis) supplemented with 0.2, 0.4, and 0.5% (w/v) porcine bile salts (Sigma). Viable cells were determined after incubation for 24 h at 37 °C by plate counting. The assays were performed in triplicate.

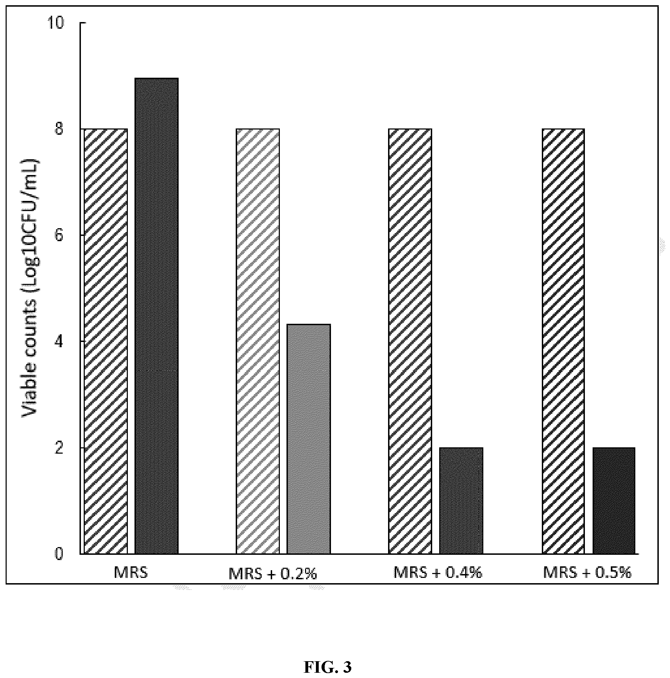

In general, *L. salivarius* PS11610 showed adequate tolerance to the different concentrations of bile salts analyzed (Figure 3). Viable counts of *L. salivarius* PS11610 reduced by 2 LOG CFU/mL when exposed to the lowest concentration of porcine bile salts (0.2%) added to the MRS broth. The effect was greater when increasing the concentration to 0.4 and 0.5% of bile salts.

### 1.7. Determination of antimicrobial spectrum

To evaluate the production of antimicrobial compounds, an overlay method described by Magnusson and Schnürer [Applied and Environmental Microbiology, 2001, 67, 1-5] was used. After the growth of the *Lactobacillus salivarius* strain on the surface of MRS agar plates at 37 °C for 24 h in aerobic conditions, the plates were overlaid with the indicator microorganism. Plates were incubated according to the appropriate condition for each indicator and the inhibition zone diameters around the streaks of *L. salivarius* PS1 1610 strain were measured. The assays were performed in triplicate.

The following bacteria were employed as indicator organisms: *Actinomyces neuii* 1-537-1 and 1-326-2, *Actinomyces urogenitalis* 1-323-81, *Aerococcus urinae* 1-278-2 and 1-265-3, *Gardnerella vaginalis* 1-265-81 and *Candida albicans* 1-534-2 from Probisearch' culture collection and originally isolated from cases of vaginal dysbiosis; *Klebsiella oxitoca* mc151, mc153 and *Streptococcus agalactiae* 30 from Probisearch' culture collection; *Escherichia coli* CECT 515, *Klebsiella pneumoniae* CECT 144, *Serratia marcescens* CECT 977, *Enterococcus faecalis* CECT 481, *Staphylococcus aureus* CECT 86 and *Staphylococcus epidermidis* CECT 231 and *Listeria innocua* CECT 910 were provided by the Spanish Collection of Type Cultures (CECT, Burjassot, Spain) and *Cronobacter sakazakii* LMG 2762 of Belgian Coordinated Collections of Microorganisms.

*L. salivarius* PS11610 showed a good antimicrobial activity against all indicator organisms used in this study (Table 3). In this sense, we observed that the *L. salivarius* PS11610 produced halos around colonies ranging from 2 mm against *Candida albicans* 1-534-2 to 50.7 mm against *Actinomyces neuii* 1-537-1. Also, the strain exhibited the highest antimicrobial activity (inhibition zone >40 mm) against microorganisms related to infections of the female genital tract: *Actinomyces neuii, Aerococcus urinae, Actinomyces urogenitalis* and *Gardnerella vaginalis.*

**Table 3. Antimicrobial activity of L. salivarius PS11610**

| **Indicator organisms** | **inhibition zone (mm)** |
|---|---|
| *Actinomyces neuii* 1-537-1 | 50.67 |
| *Actinomyces neuii* 1-326-2 | 49 |
| *Aerococcus urinae* 1-265-3 | 38.3 |
| *Aerococcus urinae* 1-278-2 | 44.7 |
| *Actinomyces urogenitalis* 1-323-81 | 38 |
| *Gardnerella vaginalis* 1-265-81 | 40 |
| *Candida albicans* 1-534-2 | 2 |
| *Klebsiella oxytoca* mc151 | 23 |
| *Klebsiella oxytoca* mc153 | 31 |
| *Streptococcus agalactiae* 30 | 12 |
| *Escherichia coli* CECT 515 | 26 |
| *Klebsiella pneumoniae* CECT 144 | 26 |
| *Serratia marcescens* CECT 977 | 30 |
| *Cronobacter sakazakii* LMG2762 | 11 |
| *Enterococcus faecalis* CECT 481 | 24.7 |
| *Staphylococcus aureus* CECT86 | 12 |
| *Staphylococcus epidermidis* CECT231 | 27 |
| *Listeria innocua* CECT 910 | 29.7 |

### 1.8. Production of lactic acid and acetic acid

Lactic acid production by the *L. salivarius* PS11610 was determined in MRS broth after 24 h at 37 °C under aerobic conditions. Cells were removed by centrifugation at 19,000×g for 20 min, and the concentration of L- and D-lactic acid in the supernatants was quantified using an enzymatic kit (Roche Diagnostics, Mannheim, Germany) following the manufacturer's instructions. The pH value of the supernatant was also measured. These assays were performed in triplicate and the values were expressed as the mean ± SD.

*L. salivarius* PS11610 produced a high concentration of L-lactic acid (9.53 ± 0.01) and decrease the pH of the culture medium until 3.96.

### 1.9. Liquid co-culture of lactobacilli and pathogens

Firstly, the correct growth of indicators in MRS media was evaluated. The co-cultures were made inoculating 1 mL of the PS11610 strain culture and 1 mL the indicator culture (DO ~ 1) in 20 mL of MRS broth. This mixture was incubated at 37 °C in aerobic condition for 17 hours. We use an initial inoculum concentration of 7 log10 cfu/mL for lactobacilli and indicator microorganism. The bacterial counts of *L*. *salivarius* PS11610 and pathogens microorganisms were made on MRS plates and BHI agar plates, respectively. Different bacteria were employed as indicator organisms, namely: *Streptococcus agalatiae* 30 and 41, *Streptococcus pyogenes* CECT 190, *Staphylococcus aureus* CECT 86, *Staphylococcus epidermidis* CECT 231, *Cronobacter sakazakii* LMG 2762, *Klebsiella oxytoca* mc151 and 153 and *Morganella morganii* 0149-10.

The co-cultures with the indicator microorganisms did not affect the viability and the growth of *L. salivarius* PS11610. However, this strain was able to decrease in 6 log units the bacterial counts of the *St. agalactiae* 30, *St. agalactiae* 41 and *St. pyogenes* CECT190. Furthermore, *L. salivarius* PS11610 was effective against *S. epidermidis* CECT231 and *S. aureus* CECT86, reducing their viable counts 2.35 and 1.48 LOG CFU, respectively. In addition, *L. salivarius* PS11610 showed good activity against Gram-negative microorganisms. It inhibit *M. morganii* 0149-10 1.78 log and 5 log units to *K. oxytoca* mc151 and *K. oxytoca* mc 153.

### 1.10. Formation of biogenic amines

The ability to form biogenic amines was assessed on decarboxylase broth prepared and adding an amino acid precursor (tyrosine, histidine, ornithine, or lysine) and a pH indicator as described before [Bover-Cid and Holzapfel, International Journal of Food Microbiology, 1999, 53, 33-41]. *L. salivarius* PS11610 was streaked onto the plates and was incubated at 37 °C under aerobic and anaerobic conditions for 4 days. A positive result was indicated by a change of the medium color to purple in response to the pH shift caused by the production of the more alkaline biogenic amine from the amino acid precursor included in the culture medium. These assays were performed by triplicate.

The PS11610 strain was not able to produce biogenic amines. Thus no allergic reactions are expected with this strain.

### Example 2. Pilot study to evaluate the effect of the strain Lactobacillus salivarius PS11610 on the microbiota of the female and male genital tract in couples with fertility problems

This study was approved by the Ethic Committee "Comité Etico de Investigación Clinica del Hospital Universitario La Paz" on the 13th of July 2018.

### 2.1. Study summary

### Primary Study objective

The main objective of this study was to investigate the effect of the new probiotic strain *Lactobacillus salivarius* PS11610 on the microbial composition of vagina, glans and semen.

### Secondary study objective(s)

The secondary objectives of this study were to know the bacterial dysbiosis rate of couples with idiopathic fertility; the pregnancy rate; the bacterial composition and the immunological profile of the uterus.

### Study product

Capsules (300 mg) comprising freeze-dried *Lactobacillus salivarius* PS11610 (about 1·10⁹ CFU) and maltodextrin as excipient.

### Summary

All couples that have initiated artificial insemination (AI) or in vitro fertilization (IVF) treatment or in waiting list for them were asked to participate in this study. The study had four visits, V1 or preselection visit where couples were recruited; V2 that took place 6 weeks ± 7 days after preselection visit, V3 that took place after 3 months ± 7 days of treatment and visit 4 that took place after 6 months ± 7 days of treatment.

At Visit 1, after verification of the inclusion and exclusion criteria and signing the informed consent, couples were supplied with all the material for the collection of the vaginal, seminal and glans sample.

Women were asked to collect and freeze (-20°C) 1 vaginal sample every week during 4 weeks (corresponding to 4 phases of the menstrual cycle). Men were asked to collect the day before the shipment of the samples to Probisearch (sponsor of this pilot study), 1 seminal sample and 1 glans sample, following the provided instructions. Once collected, they were sent by courier to Probisearch for microbiological analysis.

The criteria to determine the bacterial dysbiosis status were:
- Bacterial concentration <50 CFU in vaginal exudates.
- Lactobacilli concentration in ovulation <10² CFU.
- Concentration of corynebacteria, enterococci and/or *Staphylococcus aureus* > 10⁵ CFU.
- Presence of: *Actinomyces neuii, Gardnerella vaginalis* and/or enterobacteriaceae.

At Visit 2, 6 weeks ± 7 days after Visit 1, 15 couples with a microbiological result of dysbiosis in one of the collected samples started the intervention period. The investigator provided the probiotic that should be taken for the next 3 months. Women took one capsule of probiotic every 12 hours and men every 24 hours for the next 6 months.

Participants were given a diary and instructions on how to record the doses of the study product taken. At this visit, a uterus sample was also collected and sent to the lab for the microbiological and immunological analysis. Investigator instructed participants to not intake other probiotic supplements during their participation. Subjects were instructed to collect a vaginal sample; a semen sample and a glans sample the day before of visit 3. They were instructed to perform a blood test on the day after the visit, before starting the probiotic intake.

Those couples without bacterial dysbiosis finished their participation in the study after this visit.

At the third visit (Visit 3, 3 months ± 7 days of treatment) collected vaginal, semen and glans samples were delivered. The leftover product from the first treatment period was collected and the study product corresponding to the second supplement administration period was dispensed. Adverse Events occurring during the period between Visit 2 and Visit 3 were recorded. Participants were asked regarding the product intake compliance and to collect a vaginal sample, a semen sample and a glans sample the day before of visit 4.

During Visit 4, after 6 months ± 7 days of intervention period, collected vaginal, semen and glans samples were delivered. A uterus sample was collected and sent to the lab for the microbiological and immunological analysis. Participants returned the remaining product and the completed diary. Adverse Events occurring since Visit 3 were recorded. They were instructed to perform a blood test on the day after the visit.

In case of pregnancy during the intervention treatment, an additional visit was done upon knowing the new state. Participants collected a sample of vaginal, glans and semen the day before the visit and delivered them to the hospital. From the confirmation of the pregnancy, only the woman continued taking the probiotic once a day during the first 12 weeks of gestation. The man stopped taking the probiotic from that moment. During this visit she was provided with probiotic for the following 3 months and the previous one was collected. They were also be provided with a new product take-up compliance calendar.

Once the 12 weeks of gestation were completed, the last study visit was made and the remaining probiotic and the completed calendar were returned.

### Duration of the study

The study was conducted at the Hospital Universitario La Paz and recruitment was carried out between January 2019 and December 2019.

### Inclusion and Exclusion criteria

The inclusion criteria were:
- Couples with ages between 20 and 40
- Couples under an assisted reproduction treatment: Artificial Insemination or In vitro Fertilization
- Signature of the Informed Consent

Couples with any of the following characteristics will be excluded from the study:
- Anovulation
- Hyperprolactinemia
- Hypogonadotropic hypergonadism
- Hypergonadotropic Hypergonadism
- Hyperandrogenisms
- Polycystic ovary syndrome
- Endometriosis
- Pelvic adhesions
- Myomas, polyps and / or uterine synechia
- Diagnosis of tubal factor (hydrosalpinx, tubal obstructions)
- Low ovarian reserve
- Azoospermia
- Sperm motility (A + B) <25%
- Sperm morphology ≤2%
- With chronic diseases that cause intestinal malabsorption
- With known congenital or acquired immunodeficiency
- Obesity (IMC ≥ 30)
- Current history or diagnosis of alcohol, tobacco and drug abuse
- Uncertainty about the willingness or ability of the participants to comply with the requirements of the protocol

The inclusion criteria for the intervention treatment was to present bacterial dysbiosis in the vaginal mucosa and/or glans and/or semen.

### Sample collection and laboratory measurements

Samples collected at home by the participants were immediately frozen (-15°C to -20°C) and kept at that temperature until collected by Probisearch personnel for subsequent analysis. The uterus samples collected during visits 2 and 4 were sent to Probisearch by courier in the next hours after collection.

To characterize the bacterial composition of the vaginal smear, uterus, glans and semen and the possible changes produced after ingesting the probiotic, samples were plated in different culture media for the isolation and quantification of bacteria. Isolates were identified by MALDI-TOF mass spectrometry on a Vitek-MS ^{™} (BioMérieux, Marcy l'Etoile, France). The immune profile of the uterine samples were performed by multiplexing a Luminex^{®} 200 ^{™} (Luminexcorp, The Netherlands). The results of the analysis were sent to the investigators once they were obtained.

The uterus and plasma samples were prepared to determine the inflammation immune profile using the Bio-Plex Pro^{™} Human Inflammation Panel 1, 37-Plex that includes the following analytes: APRIL / TNFSF13, BAFF / TNFSF13B, sCD30 / TNFRSF8, sCD163, Chitinase-3-like 1, gp130 / sIL-6Rβ, IFN-α2, IFN-β, IFN-y, IL-2, sIL-6Rα, IL-8, IL-10, IL-11, IL-12 (p40), IL-12 (p70), IL-19, IL-20, IL-22, IL-26, IL-27 (p28), IL-28A / IFN-λ2, IL-29/IFN-λ1, IL-32, IL-34, IL-35, LIGHT / TNFSF14, MMP-1, MMP-2, MMP-3, Osteocalcin, Osteopontin, Pentraxin-3, sTNF-R1, sTNF-R2, TSLP, TWEAK / TNFSF12. And the MILLIPLEX^{®} MAP TGFβ1,2,3 MAGNETIC BEAD KIT to detect and quantify the three isoforms of transforming growth factor-beta (TGF-β1, TGF-β2 and TGF-β3). The analysis was carried out in duplicate on a Luminex 200 kit (Luminexcorp., The Netherlands).

### 2.2. Statistical Analysis

The data was analysed using the R software package (version 3.6.0, R project, http:// www.R-project.org).

Qualitative variables were described using absolute and relative frequencies. Quantitative variables were described using central tendency parameters (using mean, median, or mode) and dispersion (using standard deviation, the confidence interval, interquartile range, or minimum and maximum).

A result was considered statistically significant when the statistical test yields a two-sided probability of 0.05 or less.

All safety analyses were performed on the entire study population, and the efficacy analyses were carried out in the intention-to-treat population.

The primary outcome is the microbial composition of the vaginal mucosa, glans and semen. This variable was obtained as a combination of the concentrations of each of the species detected in the samples of interest, of each participating couple. For the comparison of these variables in the different times of the study GLM was used.

### 2.3. Results

### Subjects

In this study 15 couples were contacted. Of these, 14 were eligible to start with the product intake. Among them 9 completed the study and 5 couples withdrew from the study. None of the early terminations was due to and adverse effect.

The median age of men and women were very similar, 36 and 35, respectively. The minimum age of the men was 30, of the women it was 31, the maximum age of the men was 42 and of the women 38. All participants were Caucasic.

### Results of the outcome parameters

For the analysis of data, the results obtained for the 9 couples that completed the study was considered as the principal analysis.

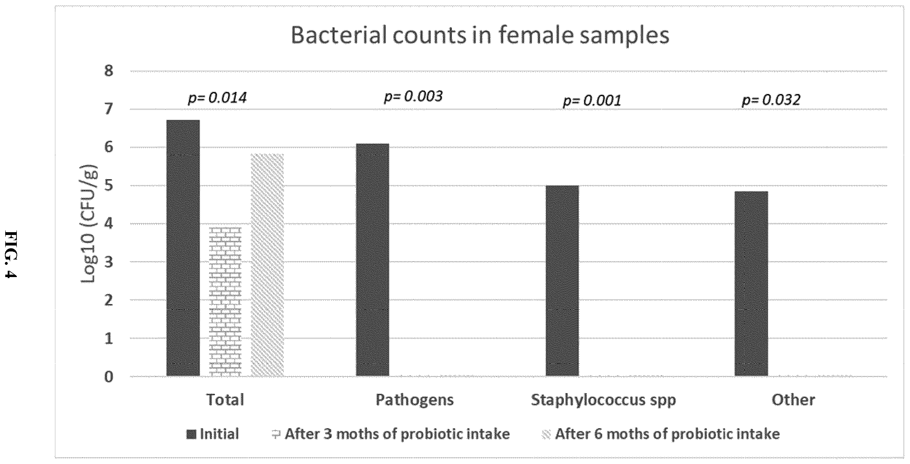

The bacterial composition of the vaginal mucosa changed statistically significant after 3 and 6 months of treatment with *L. salivarius* PS1 1610 strain. A statistically significant decrease of pathogens and staphylococci was detected in the vaginal samples (Figure 4). In the case of the male samples, a decrease of the bacterial counts was also detected although it was not statistically significant.

The criteria to determine the bacterial dysbiosis status of the couples to be eligible to start the product intake were:
- Bacterial concentration <50 CFU in vaginal swabs.
- Lactobacilli concentration in ovulation <10² CFU.
- Concentration of corynebacteria, enterococci and/or *Staphylococcus aureus*> 10⁵ CFU.
- Presence of: *Actinomyces neuii, Gardnerella vaginalis* and/or *Enterobacteriaceae.*

In table 4 the evolution of the criteria related with bacterial dysbiosis in this study is shown.

**Table 4. Number of couples with any of the dysbiosis criteria according to the protocol**

| | Visit 2 (N= 14) | Visit 3 (N= 10) | Visit 4 (N= 9) |
|---|---|---|---|
| <50 CFU in vaginal swabs | 2 | 0 | 1 |
| <100 CFU Lactobacilli in Luteus Phase (Phase III <> Ovulation) | 4 | 1 | 1 |
| Couple with counts > 10⁵ CFU | | | |
| *Corynebacterium* | 12 | 4 | 3 |
| *Enterococcus* | 4 | 2 | 1 |
| *S. aureus* | 1 | 0 | 0 |

| Detection of | | | |
|---|---|---|---|
| *Actinomyces neuii* | 4 | 2 | 0 |
| *Gardnerella vaginalis* | 6 | 0 | 1 |
| Enterobacteria | 4 | 1 | 0 |

After 3 months of treatment 50% of the couples solved the dysbiosis while the 62.5% of the couples did it after 6 months of probiotic intake (p = 0.001). Both, the total number and the mean number of dysbiosis criteria statistically decreased with a 95% signification (p < 0.030 and p < 0.034, respectively).

Regarding the bacterial dysbiosis rate of couples with idiopathic infertility, all the contacted couples that met the inclusion and exclusion criteria showed bacterial dysbiosis. In 7 of 14 (50%) couples both members showed bacterial dysbiosis while in 3 of 14 (21%) was the women who met at least one of the dysbiosis criteria and in 4 of 14 (29%) was the men who showed dysbiosis but not the women.

In this study, 4 of the 9 couples (44.44%) that finished the study became pregnant. One of them suffered an ectopic pregnancy, 1 got pregnant after 3 months of product intake, 1 got pregnant after 3 weeks of product intake, and 1 got pregnant after 6 months of product intake. Three continue with pregnancy and gave birth successfully.

In view of the above results it can be concluded that the intake for 6 months of *Lactobacillus salivarius* PS 11610 exerts a strong effect on the bacterial composition of the urogenital male and female tract and the immune status of couples with infertility caused by bacterial dysbiosis. These effects improve the pregnancy success in infertility couples.

Regarding the immunological profile of the uterus, no statistically significant changes were observed for each one of the 37 immune compounds assessed (Table 5). Despite that fact, the immune status of the uterus changed from a pro-inflammatory profile in visit 2 to a more anti-inflammatory profile after 6 months of the probiotic treatment in visit 4.

**Table 5. Immune compounds assessed in uterus samples at the beginning and at the end of the treatment.**

| | Uterus | | |
|---|---|---|---|
| | Initial (N=10) | Final (N=7) | K-W p-value |
| APRIL/TNFSF13 * | 166.20 (122.44 - 304.92) | 207.04 (139.38 - 278.41) | 0.558 |
| BAFF/TNFSF13B* | 11.58 (7.71 - 16.67) | 13.55 (9.07 - 25.59) | 0.696 |
| sCD30/TNFRSF8 * | 0.37 (0.16 - 0.55) | 0.24 (0.13 - 0.49) | 0.696 |
| sCD163^{¥} | 83.37 (55.84 - 177.63) | 89.98 (70.26 - 565.60) | 0.637 |
| Chitinase 3-like 1* | 29.11 (18.11 - 31.99) | 40.69 (40.57 - 48.35) | 0.072 |
| gp 130/sIL-6Rβ* | 96.60 (60.28 - 166.33) | 105.33 (79.42 - 148.55) | 0.922 |
| INF-α2^{¥} | 19.24 (13.82 - 39.58) | 15.04 (13.23 - 20.14) | 0.696 |
| INF-β^{¥} | 51.05 (38.47 - 72.36) | 34.75 (22.82 - 61.53) | 0.435 |
| INF-γ^{¥} | 7.31 (6.02 - 14.24) | 7.58 (5.41 - 9.89) | 0.845 |
| IL-2^{¥} | 16.74 (13.04 - 26.79) | 14.31 (13.67 - 90.24) | 0.770 |
| sIL-6Rα* | 3.18 (1.67 - 3.80) | 2.05 (1.26 - 2.75) | 0.558 |
| IL-8* | 1.72 (0.73 - 4.49) | 3.29 (1.76 - 7.18) | 0.380 |
| IL-10^{¥} | 55.78 (30.56 - 79.28) | 77.59 (38.57 - 96.44) | 0.380 |
| IL-11^{¥} | 8.28 (4.83 - 258.51) | 18.66 (4.60 - 29.65) | 1.000 |
| IL-12p40^{¥} | 34.63 (28.09 - 56.73) | 34.62 (30.59 - 35.63) | 1.000 |
| IL-12p70^{¥} | 2.04 (1.16 - 4.31) | 1.96 (1.64 - 2.60) | 0.732 |
| IL-19^{¥} | 8.22 (5.29 - 13.05) | 8.77 (4.20 - 12.88) | 0.845 |
| IL-20^{¥} | 38.92 (23.06 - 64.32) | 32.22 (24.81 - 57.55) | 0.884 |
| IL-22^{¥} | 130.15 (71.58 - 166.07) | 106.62 (73.80 - 144.78) | 0.922 |
| IL-26* | 4.90 (3.35 - 7.56) | 4.67 (3.40 - 6.68) | 0.845 |
| IL-27p28* | 0.34 (0.14 - 0.64) | 0.22 (0.20 - 0.23) | 0.958 |
| IL-28A/INF-λ2^{¥} | 53.65 (50.28 - 70.95) | 50.28 (47.90 - 52.60) | 0.403 |
| IL-29/INF-λ1* | 0.14 (0.10 - 0.30) | 0.18 (0.12 - 0.23) | 0.807 |
| IL-32* | 0.26 (0.21 - 0.28) | 0.28 (0.25 - 0.41) | 0.329 |
| IL-34* | 0.66 (0.56 - 0.88) | 0.71 (0.63 - 0.86) | 0.770 |
| IL-3 5* | 0.14 (0.09 - 0.18) | 0.12 (0.09 - 0.13) | 0.464 |
| LIGHT/TNFSF14^{¥} | 52.87 (42.01 - 67.98) | 44.17 (39.84 - 59.66) | 0.626 |
| M1VVIP-1* | 3.09 (3.04 - 6.94) | 2.39 (1.33 - 7.03) | 0.513 |
| MMP-2* | 40.52 (23.33 - 56.33) | 22.43 (16.30 - 47.85) | 0.696 |
| MMP-3* | 3.68 (2.57 - 14.85) | 3.29 (2.69 - 5.96) | 0.922 |
| Osteocalcin* | 0.76 (0.66 - 1.44) | 0.83 (0.66 - 0.92) | 0.770 |
| O steop ontin * | 22.64 (18.78 - 24.93) | 23.06 (19.32 - 30.94) | 0.906 |
| Pentraxin-3* | 10.03 (7.01 - 11.48) | 8.46 (3.93 - 12.87) | 0.626 |
| sTNF-R1* | 3.23 (1.67 - 4.46) | 2.50 (1.86 - 3.93) | 0.845 |
| sTNF-R2* | 0.66 (0.50 - 0.75) | 0.53 (0.45 - 0.70) | 0.770 |
| TSLP^{¥} | 36.74 (23.67 - 91.65) | 24.84 (21.22 - 28.03) | 0.380 |
| TWEAK/TNFSF12 * | 21.96 (13.57 - 38.08) | 8.72 (6.01 - 41.98) | 0.435 |
| TGF-β1* | 0.24 (0.11 - 0.34) | 0.25 (0.20 - 0.26) | 0.922 |
| TGF-β2* | 0.40 (0.09 - 3.21) | 0.45 (0.15 - 12.03) | 0.626 |
| TGF-β3^{¥} | 5.86 (3.03 - 9.90) | 9.51 (4.78 - 13.76) | 0.361 |

| | | | |
|---|---|---|---|
| * ng/mL ^{¥} pg/mL | | | |

Regarding the systemic immunological profile, there were statistically significant changes in the general immune status of the couples. At the beginning of the study, both male and female showed a pro-inflammatory profile that became anti-inflammatory after 6 months of the probiotic treatment (Table 6). In male plasma samples INF-α2, IL-12p40, IL-22, IL-28A/INF-λ2, IL-29/INF-λ1, IL-34 and TGF-β1 decreased significantly after 6 months of intake of L. salivarius PS11610. In female samples IL-12p40, IL-12p70, IL-22, IL-27p28, IL-29/INF-λ1, IL-34, IL-35, Osteocalcin, Pentraxin-3, TGF-β1 and TGF-β2 decreased in plasma at the end of the treatment statistically significant. (Table 6). Comparison of the immune profile of female samples (uterus and plasma) showed a high difference in the concentration of the numerous immune compounds highlighting differences between systemic immune response and local mucosal immune response (Table 7).

In view of the above immune profile results it can be concluded that the intake for 6 months of *Lactobacillus salivarius* PS11610 produces a change in the immune uterus profile from a pro-inflammatory profile to an anti-inflammatory profile. The intake for 6 months of *Lactobacillus salivarius* PS 1161 0 produces a change in the immune plasma profile both in men and women from a pro-inflammatory profile to an anti-inflammatory profile.

**Table 6. Immune compounds present in plasma samples at the beginning and at the end of the treatment**

| | Plasma | | | | | |
|---|---|---|---|---|---|---|
| | Male | | | Female | | |
| | Initial (N=9) | Final (N=7) | K-W p-value | Initial (N=9) | Final (N=7) | K-W p-value |
| APRIL/TNFS13 * | 288.31 (207.49 - 343.84) | 252.85 (217.01 - 330.74) | 0.711 | 324.58 (273.62 - 366.24) | 289.81 (264.86-315.35) | 0.427 |
| BAFF/TNFSF13B* | 12.96 (11.93 - 16.93) | 13.64 (10.14 - 15.31) | 0.711 | 11.10 (10.74-15.84) | 11.39 (10.50 - 12.20) | 0.791 |
| sCD3 0/TNFRSF 8 * | 0.78 (0.67 - 1.19) | 0.81 (0.64 - 1.07) | 0.958 | 0.89 (0.72 - 1.05) | 0.93 (0.81 - 1.40) | 0.560 |
| sCD163^{¥} | 161.74 (113.01 - 177.83) | 120.77 (90.79 - 226.67) | 0.560 | 136.56 (81.13 - 188.25) | 177.30(127.12-192.38) | 0.427 |
| Chitinase 3-like 1* | 14.32 (13.48 - 15.04) | 8.87 (6.84 - 9.48) | 0.186 | 9.28 (6.75 - 13.82) | 13.03 (11.00 - 14.88) | 0.224 |
| gp 130/sIL-6Rβ* | 127.64 (114.23 - 137.84) | 136.87 (103.84 - 169.47) | 0.958 | 104.58 (95.21 - 146.74) | 111.55 (98.45 - 154.48) | 0.491 |
| INF-α2^{¥} | 36.34 (31.56 - 50.52) | 23.40 (17.59 - 34.50) | 0.059 | 36.27 (28.09 - 44.58) | 32.77 (27.50 - 36.32) | 0.649 |
| INF-β^{¥} | 49.87 (43.77 - 61.08) | 47.84 (42.29 - 50.36) | 0.368 | 50.87 (44.84 - 57.00) | 46.80 (43.27 - 49.88) | 0.186 |
| INF-γ^{¥} | 16.26 (14.10 - 22.73) | 15.18 (10.87 - 19.49) | 0.479 | 18.42 (14.10 - 20.57) | 14.10(9.26-16.25) | 0.202 |
| IL-2^{¥} | 22.47 (20.68 - 28.59) | 24.67 (19.13 - 28.92) | 0.932 | 23.47 (18.68 - 26.24) | 21.59 (17.85 - 25.33) | 0.737 |
| sIL-6Rα* | 12.56 (11.22 - 13.73) | 11.68 (11.39 - 14.25) | 0.791 | 14.21 (12.60-15.46) | 12.01 (10.56 - 14.84) | 0.368 |
| IL-8* | 0.03 (0.03 - 0.04) | 0.03 (0.02 - 0.04) | 0.643 | 0.02 (0.02 - 0.03) | 0.02 (0.02 - 0.02) | 0.221 |
| IL-10^{¥} | 265.73 (230.16 - 284.87) | 270.17 (245.13 - 307.61) | 0.427 | 262.92 (183.12 - 308.64) | 270.09 (216.17 - 342.86) | 0.560 |
| IL-11^{¥} | 7.03 (6.14 - 12.57) | 6.95 (6.46 - 7.68) | 0.634 | 5.97 (2.80 - 8.07) | 4.15 (3.28 - 5.08) | 0.427 |
| IL-12p40^{¥} | 48.44 (48.36 - 53.55) | 43.22 (32.97 - 48.36) | 0.078 | 53.59 (48.36 - 58.84) | 38.03 (32.93 - 43.24) | 0.039 |
| IL-12p70^{¥} | 6.55 (3.98 - 9.78) | 3.98 (3.66 - 4.30) | 0.643 | 4.62 (4.62 - 5.91) | 2.39 (2.22 - 2.55) | 0.060 |
| IL-19^{¥} | 0.14 (0.12 - 0.16) | 0.13 (0.12 - 0.14) | 0.560 | 0.13 (0.13 - 0.15) | 0.11 (0.09 - 0.14) | 0.368 |
| IL-20^{¥} | 37.56 (20.01 - 45.60) | 33.65 (24.83 - 75.81) | 0.958 | 24.14 (18.15 - 66.20) | 18.47 (18.15 - 32.95) | 0.637 |
| IL-22^{¥} | 48.40 (20.33 - 89.64) | NA | <0.001 | 53.06 (25.12 - 91.09) | NA | <0.001 |
| IL-26* | 4.33 (4.12 - 4.75) | 5.38 (4.64 - 5.80) | 0.427 | 4.75 (3.49 - 6.13) | 4.75 (3.33 - 4.91) | 0.751 |
| IL-27p28* | 0.27 (0.22 - 0.36) | 0.16 (0.16 - 0.16) | 0.480 | 0.23 (0.12 - 0.24) | NA | <0.001 |
| IL-28A/INF-λ2^{¥} | 95.37 (86.24 - 154.53) | 55.26 (42.40 - 91.07) | 0.048 | 91.39 (70.95 - 122.50) | 72.96 (59.68 - 99.82) | 0.297 |
| IL-29/INF-λ1* | 0.19 (0.14 - 0.24) | 0.09 (0.07 - 0.13) | 0.009 | 0.18 (0.11 - 0.29) | 0.10 (0.08 - 0.12) | 0.080 |
| IL-32* | 0.77 (0.71 - 1.09) | 0.74 (0.73 - 0.87) | 0.791 | 0.88 (0.76 - 0.93) | 0.88 (0.76 - 1.12) | 0.791 |
| IL-34* | 1.19 (0.75 - 1.38) | 0.75 (0.56 - 0.86) | 0.050 | 0.93 (0.73 - 1.06) | 0.59 (0.57 - 0.65) | 0.050 |
| IL-3 5 * | 0.21 (0.19 - 0.29) | 0.20 (0.17 - 0.27) | 0.491 | 0.23 (0.20 - 0.26) | 0.18 (0.17 - 0.21) | 0.044 |
| LIGHT/TNFSF14^{¥} | 87.98 (87.98 - 87.98) | NA | na | 45.10 (30.12-60.07) | 8.82 (8.82 - 8.82) | 0.221 |
| MMP-1* | 1.15 (1.15 - 1.15) | NA | na | NA | NA | |
| MMP-2* | 40.44 (29.48 - 53.01) | 61.09 (36.38 - 62.69) | 0.224 | 39.58 (28.41 - 43.68) | 49.83 (31.49 - 60.85) | 0.224 |
| MMP-3* | 33.30 (16.23 - 40.47) | 23.53 (19.05 - 35.96) | 0.874 | 25.11 (16.42 - 31.93) | 16.86 (15.81 - 38.97) | 0.711 |
| Osteocalcin* | 2.24 (2.08 - 3.02) | 2.86 (1.95 - 2.94) | 0.711 | 2.55 (2.32 - 3.02) | 2.40 (1.73 - 2.55) | 0.080 |
| Osteopontin* | 32.40 (31.00 - 35.10) | 37.90 (30.83 - 39.84) | 0.186 | 41.72 (28.87 - 42.54) | 36.55 (31.52 - 39.54) | 0.560 |
| Pentraxin-3* | 23.80 (16.80 - 30.31) | 25.84 (19.52 - 35.59) | 0.711 | 25.26 (21.10 - 26.23) | 18.31 (16.18 - 20.58) | 0.064 |
| sTNF-R1* | 0.60 (0.57 - 0.68) | 0.57 (0.53 - 0.58) | 0.186 | 0.53 (0.51 - 0.67) | 0.66 (0.57 - 0.76) | 0.186 |
| sTNF-R2* | 0.84 (0.78 - 0.94) | 0.74 (0.73 - 0.95) | 0.427 | 0.87 (0.76 - 0.91) | 0.92 (0.83 - 0.98) | 0.315 |
| TSLP^{¥} | 69.06 (54.68 - 77.29) | 54.68 (46.33 - 66.45) | 0.152 | 64.90 (51.45 - 76.05) | 53.09 (47.49 - 67.41) | 0.491 |
| TWEAK/TNFSF12* | 3.66 (3.41 - 3.75) | 3.57 (3.17 - 3.98) | 0.874 | 4.21 (3.78 - 4.91) | 3.91 (3.45 - 4.35) | 0.368 |
| TGF-β1* | 6.61 (3.45 - 10.66) | 1.94 (1.35 - 3.09) | 0.023 | 5.13 (3.03 - 6.58) | 1.83 (1.27 - 3.50) | 0.050 |
| TGF-β2* | 0.45 (0.17 - 0.47) | 0.08 (0.07 - 0.15) | 0.128 | 0.23 (0.12 - 0.30) | 0.07 (0.05 - 0.16) | 0.039 |
| TGF-β3^{¥} | NA | NA | na | NA | NA | na |

| | | | | | | |
|---|---|---|---|---|---|---|
| * ng/mL ^{¥} pg/mL | | | | | | |

**Table 7. Immune profile of female samples (plasma and uterus) at the beginning and at the end of the treatment.**

| | Plasma | | | Uterus | | | | |
|---|---|---|---|---|---|---|---|---|
| | Initial | Final | K-W p-value | Initial | Final | K-W p-value | k-w p-value plasma female vs uterus initial | k-w p-value plasma female vs uterus final |
| APRIL/TNFSF13* | 324.58 (273.62 - 366.24) | 289.81 (264.86-315.35) | 0.427 | 166.20 (122.44 - 304.92) | 207.04 (139.38 - 278.41) | 0.558 | 0.102 | 0.110 |
| BAFF/TNFSF13B* | 11.10 (10.74 - 15.84) | 11.39 (10.50 - 12.20) | 0.791 | 11.58 (7.71 - 16.67) | 13.55 (9.07 - 25.59) | 0.696 | 0.568 | 0.338 |
| sCD3 0/TNFRSF 8 * | 0.89 (0.72 - 1.05) | 0.93 (0.81 - 1.40) | 0.560 | 0.37 (0.16 - 0.55) | 0.24 (0.13 - 0.49) | 0.696 | 0.022 | 0.025 |
| sCD163^{¥} (pg/mL) | 136.56 (81.13 - 188.25) | 177.30 (127.12 - 192.38) | 0.427 | 83.37 (55.84 - 177.63) | 89.98 (70.26 - 565.60) | 0.637 | 0.401 | 0.475 |
| Chitinase 3-like 1* | 9.28 (6.75 - 13.82) | 13.03 (11.00 - 14.88) | 0.224 | 29.11 (18.11 - 31.99) | 40.69 (40.57 - 48.35) | 0.072 | 0.012 | 0.004 |
| gp 130/sIL-6Rβ* | 104.58 (95.21 - 146.74) | 111.55 (98.45 - 154.48) | 0.491 | 96.60 (60.28 - 166.33) | 105.33 (79.42 - 148.55) | 0.922 | 0.624 | 0.565 |
| INF-α2^{¥} | 36.27 (28.09 - 44.58) | 32.77 (27.50 - 36.32) | 0.649 | 19.24 (13.82 - 39.58) | 15.04 (13.23 - 20.14) | 0.696 | 0.181 | 0.045 |
| INF-β^{¥} | 50.87 (44.84 - 57.00) | 46.80 (43.27 - 49.88) | 0.186 | 51.05 (38.47-72.36) | 34.75 (22.82 - 61.53) | 0.435 | 1.000 | 0.749 |
| INF-γ^{¥} | 18.42 (14.10 - 20.57) | 14.10 (9.26 - 16.25) | 0.202 | 7.31 (6.02 - 14.24) | 7.58 (5.41 - 9.89) | 0.845 | 0.072 | 0.084 |
| IL-2^{¥} | 23.47 (18.68 - 26.24) | 21.59 (17.85 - 25.33) | 0.737 | 16.74 (13.04 - 26.79) | 14.31 (13.67 - 90.24) | 0.770 | 0.232 | 0.769 |
| sIL-6Rα* | 14.21 (12.60 - 15.46) | 12.01 (10.56 - 14.84) | 0.368 | 3.18 (1.67 - 3.80) | 2.05 (1.26 - 2.75) | 0.558 | <0.001 | 0.002 |
| IL-8* | 0.02 (0.02 - 0.03) | 0.02 (0.02 - 0.02) | 0.221 | 1.72 (0.73 - 4.49) | 3.29(1.76-7.18) | 0.380 | 0.032 | 0.127 |
| IL-10^{¥} | 262.92 (183.12 - 308.64) | 270.09 (216.17 - 342.86) | 0.560 | 55.78 (30.56 - 79.28) | 77.59 (38.57 - 96.44) | 0.380 | <0.001 | 0.002 |
| IL-11^{¥} | 5.97 (2.80 - 8.07) | 4.15 (3.28 - 5.08) | 0.427 | 8.28 (4.83 - 258.51) | 18.66 (4.60-29.65) | 1.000 | 0.221 | 0.142 |
| IL-12p40^{¥} | 53.59 (48.36 - 58.84) | 38.03 (32.93 - 43.24) | 0.039 | 34.63 (28.09 - 56.73) | 34.62 (30.59 - 35.63) | 1.000 | 0.165 | 0.277 |
| IL-12p70^{¥} | 4.62 (4.62 - 5.91) | 2.39 (2.22 - 2.55) | 0.060 | 2.04 (1.16 - 4.31) | 1.96 (1.64 - 2.60) | 0.732 | 0.119 | 0.558 |
| IL-19^{¥} | 0.13 (0.13 - 0.15) | 0.11 (0.09 - 0.14) | 0.368 | 8.22 (5.29 - 13.05) | 8.77 (4.20 - 12.88) | 0.845 | <0.001 | 0.002 |
| IL-20^{¥} | 24.14 (18.15 - 66.20) | 18.47 (18.15 - 32.95) | 0.637 | 38.92 (23.06 - 64.32) | 32.22 (24.81 - 57.55) | 0.884 | 0.514 | 0.153 |
| IL-22^{¥} | 53.06 (25.12 - 91.09) | NA | <0.001 | 130.15 (71.58 - 166.07) | 106.62 (73.80 - 144.78) | 0.922 | 0.089 | <0.001 |
| IL-26* | 4.75 (3.49 - 6.13) | 4.75 (3.33 - 4.91) | 0.751 | 4.90 (3.35 - 7.56) | 4.67 (3.40 - 6.68) | 0.845 | 0.624 | 0.749 |
| IL-27p28* | 0.23 (0.12 - 0.24) | NA | <0.001 | 0.34 (0.14 - 0.64) | 0.22 (0.20 - 0.23) | 0.958 | 0.317 | <0.001 |
| IL-28A/INF-λ2^{¥} | 91.39 (70.95 - 122.50) | 72.96 (59.68 - 99.82) | 0.297 | 53.65 (50.28 - 70.95) | 50.28 (47.90 - 52.60) | 0.403 | 0.050 | 0.197 |
| IL-29/INF-λ1* | 0.18 (0.11 - 0.29) | 0.10 (0.08 - 0.12) | 0.080 | 0.14 (0.10-0.30) | 0.18 (0.12-0.23) | 0.807 | 0.744 | 0.142 |
| IL-32* | 0.88 (0.76 - 0.93) | 0.88 (0.76 - 1.12) | 0.791 | 0.26 (0.21 - 0.28) | 0.28 (0.25 - 0.41) | 0.329 | <0.001 | 0.002 |
| IL-34* | 0.93 (0.73 - 1.06) | 0.59 (0.57 - 0.65) | 0.050 | 0.66 (0.56 - 0.88) | 0.71 (0.63 - 0.86) | 0.770 | 0.165 | 0.180 |
| IL-35* | 0.23 (0.20 - 0.26) | 0.18 (0.17-0.21) | 0.044 | 0.14 (0.09 - 0.18) | 0.12 (0.09 - 0.13) | 0.464 | 0.050 | 0.064 |
| LIGHT/TNFSF14^{¥} | 45.10 (30.12 - 60.07) | 8.82 (8.82 - 8.82) | 0.221 | 52.87 (42.01 - 67.98) | 44.17 (39.84 - 59.66) | 0.626 | 0.667 | 0.127 |
| MMP-1* | NA | NA | | 3.09 (3.04 - 6.94) | 2.39 (1.33 - 7.03) | 0.513 | <0.001 | <0.001 |
| MMP-2* | 39.58 (28.41 - 43.68) | 49.83 (31.49 - 60.85) | 0.224 | 40.52 (23.33 - 56.33) | 22.43 (16.30 - 47.85) | 0.696 | 0.870 | 0.225 |
| MMP-3* | 25.11 (16.42 - 31.93) | 16.86 (15.81 - 38.97) | 0.711 | 3.68 (2.57 - 14.85) | 3.29 (2.69-5.96) | 0.922 | 0.034 | 0.025 |
| Osteocalcin* | 2.55 (2.32 - 3.02) | 2.40 (1.73 - 2.55) | 0.080 | 0.76 (0.66 - 1.44) | 0.83 (0.66 - 0.92) | 0.770 | 0.003 | 0.025 |
| Osteopontin * | 41.72 (28.87 - 42.54) | 36.55 (31.52 - 39.54) | 0.560 | 22.64 (18.78 - 24.93) | 23.06 (19.32 - 30.94) | 0.906 | 0.004 | 0.198 |
| Pentraxin-3* | 25.26 (21.10 - 26.23) | 18.31 (16.18 - 20.58) | 0.064 | 10.03 (7.01 - 11.48) | 8.46 (3.93 - 12.87) | 0.626 | <0.001 | 0.025 |
| sTNF-R1* | 0.53 (0.51 - 0.67) | 0.66 (0.57 - 0.76) | 0.186 | 3.23 (1.67 - 4.46) | 2.50 (1.86 - 3.93) | 0.845 | 0.002 | 0.002 |
| sTNF-R2* | 0.87 (0.76 - 0.91) | 0.92 (0.83 - 0.98) | 0.315 | 0.66 (0.50 - 0.75) | 0.53 (0.45 - 0.70) | 0.770 | 0.102 | 0.048 |
| TSLP^{¥} | 64.90 (51.45 - 76.05) | 53.09 (47.49 - 67.41) | 0.491 | 36.74 (23.67 - 91.65) | 24.84 (21.22 - 28.03) | 0.380 | 0.369 | 0.025 |
| TWEAK/TNFSF12* | 4.21 (3.78 - 4.91) | 3.91 (3.45 - 4.35) | 0.368 | 21.96 (13.57 - 38.08) | 8.72 (6.01 - 41.98) | 0.435 | 0.001 | 0.018 |
| TGF-β1* | 5.13 (3.03 - 6.58) | 1.83 (1.27 - 3.50) | 0.050 | 0.24 (0.11 - 0.34) | 0.25 (0.20 - 0.26) | 0.922 | <0.001 | 0.002 |
| TGF-β2* | 0.23 (0.12 - 0.30) | 0.07 (0.05 - 0.16) | 0.039 | 0.40 (0.09 - 3.21) | 0.45 (0.15 - 12.03) | 0.626 | 0.514 | 0.110 |
| TGF-β3^{¥} | NA | NA | na | 5.86 (3.03 - 9.90) | 9.51 (4.78-13.76) | 0.361 | <0.001 | <0.001 |

| | | | | | | | | |
|---|---|---|---|---|---|---|---|---|
| * ng/mL ^{¥} pg/mL | | | | | | | | |

### Example 3: Antimicrobial activity of Lactobacillus salivarius strains

To evaluate the production of antimicrobial compounds, an overlay method described by Magnusson and Schnürer [Applied and Environmental Microbiology, 2001, 67, 1-5] was used. After the growth of the corresponding *Lactobacillus salivarius* strains (*L. salivarius PS11610 of the invention, L. salivarius PS11603* and *L. salivarius PS7* from Probisearch's culture collection and available from the Spanish Collection of Type Cultures (CECT 9526, CECT 9527 and CECT 9422, respectively)) on the surface of MRS agar plates at 37 °C for 24 h in aerobic conditions, the plates were overlaid with the indicator microorganism. Plates were incubated according to the appropriate condition for each indicator and the inhibition zone diameters around the streaks of the corresponding *L*. *salivarius* strain were measured. The assays were performed in triplicate.

The following bacteria were employed as indicator organisms: *Actinomyces neuii* 1-537-1 and 1-326-2, *Actinomyces urogenitalis* 1-323-81, *Aerococcus urinae* 1-278-2 and 1-265-3, and *Gardnerella vaginalis* 1-265-81 from Probisearch' culture collection and originally isolated from cases of vaginal dysbiosis; and *Enterococcus faecalis* CECT 481 was provided by the Spanish Collection of Type Cultures (CECT, Burjassot, Spain).

*L. salivarius* PS11610 showed a higher antimicrobial activity against indicator organisms used in this study (Table 8) that the other tested *L. salivarius* strains.

**Table 8. Antimicrobial activity of L. salivarius strains. The results are expressed as the inhibition zone in mm.**

| **Indicator organisms** | ***L. salivarius* PS11610** | ***L. salivarius* PS11603** | ***L. salivarius* PS7** |
|---|---|---|---|
| *Actinomyces neuii* 1-537-1 | 51 | 31 | 33 |
| *Actinomyces neuii* 1-326-2 | 49 | 26 | 38 |
| *Aerococcus urinae* 1-278-2 | 45 | 18 | 42 |
| *Aerococcus urinae* 1-265-3 | 38 | 21 | 39 |
| *Actinomyces urogenitalis* 1-323-81 | 38 | 22 | 30 |
| *Gardnerella vaginalis* 1-265-81 | 40 | 29 | 35 |
| *Enterococcus faecalis* CECT 481 | 25 | 20 | 23 |

## Claims

1. A strain of *Lactobacillus salivarius* PS1 1610 or a variant thereof having at least 99% identity with the 16S rRNA sequence of this strain, wherein the variant strain retains or further improves the activity of the strain *Lactobacillus salivarius* PS11610 in one or more of the following properties:
- production of lactic acid in MRS broth after 24 h at 37 °C under anaerobic conditions;
- tolerance to pH in the range of 3 to 6; and
- tolerance to bile salts.

2. The strain as defined in claim 1, wherein the strain is *Lactobacillus salivarius* PS11610.

3. The strain as defined in any of claims 1 or 2, wherein the strain is alive.

4. A composition comprising a strain as defined in any of claims 1 to 3.

5. The composition according to claim 4, which comprises from about 10⁶ CFU to about 10¹² CFU of the *Lactobacillus salivarius* strain.

6. The composition according to any of claims 4 or 5, which further comprises a pharmaceutically acceptable excipient.

7. The composition according to claim 6, wherein the pharmaceutically acceptable excipient is maltodextrin.

8. The composition according to any of claims 4 to 7, which is in the form of an oral formulation, preferably a capsule.

9. Foodstuff comprising the composition according to any of claims 4 or 5.

10. A strain as defined in any of claims 1 to 3, a composition as defined in any of claims 4 to 8 or a foodstuff as defined in claim 9, for use as a probiotic or for use as a therapeutic agent.

11. A strain as defined in any of claims 1 to 3, a composition as defined in any of claims 4 to 8 or a foodstuff as defined in claim 9, for use in the prevention and/or treatment of urogenital dysbiosis.

12. A strain as defined in any of claims 1 to 3, a composition as defined in any of claims 4 to 8 or a foodstuff as defined in claim 9, for use in the prevention and/or treatment of bacterial vaginosis and vaginitis.

13. A strain as defined in any of claims 1 to 3, a composition as defined in any of claims 4 to 8 or a foodstuff as defined in claim 9, for use in the treatment of infertility in a subject suffering from urogenital bacterial dysbiosis.

14. A strain as defined in any of claims 1 to 3, a composition as defined in any of claims 4 to 8 or a foodstuff as defined in claim 9, for use in the prevention of miscarriage in the first trimester of pregnancy in *in vitro* fertilization.

15. A strain as defined in any of claims 1 to 3, a composition as defined in any of claims 4 to 8 or a foodstuff as defined in claim 9, for use in reducing pathogenic bacteria in the urogenital tract.

## Patentansprüche

1. Stamm von *Lactobacillus salivarius* PS11610 oder eine Variante davon mit mindestens 99% Identität mit der 16S rRNA-Sequenz dieses Stammes, wobei der Variantenstamm die Aktivität des Stammes *Lactobacillus salivarius* PS11610 in einer oder mehreren der folgenden Eigenschaften beibehält oder verbessert:
- Produktion von Milchsäure in MRS-Bouillon nach 24 Stunden bei 37 °C unter anaeroben Bedingungen;
- Toleranz gegenüber einem pH-Wert im Bereich von 3 bis 6; und
- Toleranz gegenüber Gallensalzen.

2. Stamm nach Anspruch 1, wobei der Stamm *Lactobacillus salivarius* PS11610 ist.

3. Stamm nach einem der Ansprüche 1 oder 2, wobei der Stamm lebend ist.

4. Zusammensetzung, die einen in einem der Ansprüche 1 bis 3 definierten Stamm aufweist.

5. Zusammensetzung nach Anspruch 4, die von etwa 10⁶ KbE bis etwa 10¹² KbE des *Lactobacillus* salivarius-Stammes aufweist.

6. Zusammensetzung nach einem der Ansprüche 4 oder 5, die außerdem einen pharmazeutisch verträglichen Hilfsstoff aufweist.

7. Zusammensetzung nach Anspruch 6, wobei der pharmazeutisch verträgliche Hilfsstoff Maltodextrin ist.

8. Zusammensetzung nach einem der Ansprüche 4 bis 7, die die Form einer oralen Formulierung, vorzugsweise einer Kapsel, aufweist.

9. Lebensmittel, aufweisend die Zusammensetzung nach einem der Ansprüche 4 oder 5.

10. Stamm, wie in einem der Ansprüche 1 bis 3 definiert, eine Zusammensetzung, wie in einem der Ansprüche 4 bis 8 definiert, oder ein Lebensmittel, wie in Anspruch 9 definiert, zur Verwendung als Probiotikum oder zur Verwendung als therapeutisches Mittel.

11. Stamm, wie in einem der Ansprüche 1 bis 3 definiert, eine Zusammensetzung, wie in einem der Ansprüche 4 bis 8 definiert, oder ein Lebensmittel, wie in Anspruch 9 definiert, zur Verwendung bei der Prävention und/oder Behandlung von urogenitaler Dysbiose.

12. Stamm, wie in einem der Ansprüche 1 bis 3 definiert, eine Zusammensetzung, wie in einem der Ansprüche 4 bis 8 definiert, oder ein Lebensmittel, wie in Anspruch 9 definiert, zur Verwendung bei der Prävention und/oder Behandlung von bakterieller Vaginose und Vaginitis.

13. Stamm, wie in einem der Ansprüche 1 bis 3 definiert, eine Zusammensetzung, wie in einem der Ansprüche 4 bis 8 definiert, oder ein Lebensmittel, wie in Anspruch 9 definiert, zur Verwendung bei der Behandlung von Unfruchtbarkeit bei einem Patienten, der an urogenitaler bakterieller Dysbiose leidet.

14. Stamm, wie in einem der Ansprüche 1 bis 3 definiert, eine Zusammensetzung, wie in einem der Ansprüche 4 bis 8 definiert, oder ein Lebensmittel, wie in Anspruch 9 definiert, zur Verwendung bei der Prävention von Fehlgeburten im ersten Trimester der Schwangerschaft bei einer In-vitro-Fertilisation.

15. Stamm, wie in einem der Ansprüche 1 bis 3 definiert, eine Zusammensetzung, wie in einem der Ansprüche 4 bis 8 definiert, oder ein Lebensmittel, wie in Anspruch 9 definiert, zur Verwendung bei der Reduzierung pathogener Bakterien im Urogenitaltrakt.

## Revendications

1. Une souche de *Lactobacillus salivarius* PS 11610 ou un variant de celle-ci ayant au moins 99 % d'identité avec la séquence d'ARNr 16S de cette souche, la souche variante conservant ou améliorant encore l'activité de la souche *Lactobacillus salivarius* PS11610 pour ce qui est d'une de ou plusieurs des propriétés suivantes :
- production d'acide lactique dans un bouillon MRS après 24 h à 37°C en conditions anaérobies ;
- tolérance au pH compris entre 3 et 6 ; et
- tolérance aux sels biliaires.

2. La souche telle que définie dans la revendication 1, **caractérisée en ce que** la souche est *Lactobacillus salivarius* PS 11610.

3. La souche telle que définie dans l'une quelconque des revendications 1 ou 2, la souche étant vivante.

4. Une composition comprenant une souche telle que définie dans l'une quelconque des revendications 1 à 3.

5. La composition selon la revendication 4, qui comprend d'environ 10⁶ CFU à environ 10¹² CFU de la souche *Lactobacillus salivarius.*

6. La composition selon l'une quelconque des revendications 4 ou 5, qui comprend en outre un excipient pharmaceutiquement acceptable.

7. La composition selon la revendication 6, dans laquelle l'excipient pharmaceutiquement acceptable est la maltodextrine.

8. La composition selon l'une quelconque des revendications 4 à 7, qui se présente sous la forme d'une formulation orale, de préférence d'une gélule.

9. Produit alimentaire comprenant la composition selon l'une quelconque des revendications 4 ou 5.

10. Une souche telle que définie dans l'une quelconque des revendications 1 à 3, une composition telle que définie dans l'une quelconque des revendications 4 à 8 ou un produit alimentaire tel que défini dans la revendication 9, pour une utilisation en tant que probiotique ou pour une utilisation en tant qu'agent thérapeutique.

11. Une souche telle que définie dans l'une quelconque des revendications 1 à 3, une composition telle que définie dans l'une quelconque des revendications 4 à 8 ou un produit alimentaire tel que défini dans la revendication 9, pour une utilisation dans la prévention et/ou le traitement de la dysbiose urogénitale.

12. Une souche telle que définie dans l'une quelconque des revendications 1 à 3, une composition telle que définie dans l'une quelconque des revendications 4 à 8 ou un produit alimentaire tel que défini dans la revendication 9, pour une utilisation dans la prévention et/ou le traitement de la vaginose et de la vaginite bactériennes.

13. Une souche telle que définie dans l'une quelconque des revendications 1 à 3, une composition telle que définie dans l'une quelconque des revendications 4 à 8 ou un produit alimentaire tel que défini à la revendication 9, pour une utilisation dans le traitement de l'infertilité chez un sujet souffrant de dysbiose bactérienne urogénitale.

14. Une souche telle que définie dans l'une quelconque des revendications 1 à 3, une composition telle que définie dans l'une quelconque des revendications 4 à 8 ou un produit alimentaire tel que défini dans la revendication 9, pour une utilisation dans la prévention des fausses couches au cours du premier trimestre de la grossesse par fertilisation *in vitro.*

15. Une souche telle que définie dans l'une quelconque des revendications 1 à 3, une composition telle que définie dans l'une quelconque des revendications 4 à 8 ou un produit alimentaire tel que défini dans la revendication 9, pour une utilisation dans la réduction des bactéries pathogènes dans le tractus urogénital.
